(19) Europäisches Patentamt / European Patent Office / Office européen des brevets

(11) **EP 4 159 760 A1**

(12) **EUROPEAN PATENT APPLICATION**
published in accordance with Art. 153(4) EPC

(43) Date of publication:
**05.04.2023 Bulletin 2023/14**

(21) Application number: **21818442.2**

(22) Date of filing: **12.04.2021**

(51) International Patent Classification (IPC):
*C07K 16/28* (2006.01)    *A61P 35/00* (2006.01)
*G01N 33/563* (2006.01)    *G01N 33/574* (2006.01)
*A61K 39/00* (2006.01)

(52) Cooperative Patent Classification (CPC):
**A61K 39/00; A61P 35/00; C07K 16/28;**
**G01N 33/563; G01N 33/574**

(86) International application number:
**PCT/KR2021/004568**

(87) International publication number:
**WO 2021/246637 (09.12.2021 Gazette 2021/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.06.2020 KR 20200065666**
           **07.12.2020 KR 20200169499**

(71) Applicant: **Innobation Bio Co., Ltd.**
**Seoul 03929 (KR)**

(72) Inventors:
• **KIM, Kitae**
**Seoul 06148 (KR)**
• **KIM, Seungkoo**
**Yongin-si, Gyeonggi-do 16865 (KR)**
• **LEE, Byungeun**
**Seoul 06148 (KR)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

(54) **ANTIBODY SPECIFIC TO CD22, AND USE THEREOF**

(57) The present invention relates to an antibody specific for CD22 and uses thereof, and more particularly to an antibody that specifically binds to CD22, a chimeric antigen receptor comprising the antibody, a CAR-T cell expressing the chimeric antigen receptor, and a pharmaceutical composition for preventing or treating diseases mediated by cells expressing CD22 including the same.

It was confirmed that the antibody selected in the present invention specifically recognized CD22-expressing cells, and the chimeric antigen receptor (CAR) and CAR-T cells targeting CD22 using the CD22-specific antibody not only effectively bound to CD22, but also activated CAR-T cells bound to CD22. In addition, since it was confirmed that the CAR-T cells of the present invention effectively kill CD22-expressing cells, the CD22-specific antibody, the chimeric antigen receptor targeting CD22, and CAR-T cells of the present invention can be usefully used as a composition for preventing or treating diseases relating to CD22 expression.

[Fig. 1]

**Description**

[Technical field]

The present invention relates to an antibody specific for CD22 and uses thereof, and more particularly to an antibody that specifically binds to CD22, a chimeric antigen receptor comprising the antibody, a CAR-T cell expressing the chimeric antigen receptor, and a pharmaceutical composition for preventing or treating diseases mediated by cells expressing CD22 comprising the same.

[Background Art]

CD22 is a 135 kDa membrane glycoprotein belonging to a family of sialic acid-binding proteins called sialoadhesins. CD22 is detected in the cytoplasm at the beginning of B cell development, appears on the cell surface simultaneously with IgD, and is found in most mature B cells. When B cells are activated, their expression increases. It has been reported that CD22 is lost at the end of differentiation and is generally absent in plasma cells.
There are two isoforms of human CD22. The dominant form (CD22β) contains seven immunoglobulin-like (Ig-like) domains in the extracellular region. The CD22α mutant lacks Ig-like domain 4 and may have a truncated cytoplasmic domain. Antibodies that block the binding of CD22 to monocytes, neutrophils, lymphocytes and erythrocytes have been observed to bind within the first or second Ig-like domain.
In the cytoplasmic domain of CD22, tyrosine is phosphorylated upon binding to the B-cell antigen receptor and binds to Lyk, Syk and phosphatidylinositol 3-kinase. The function of CD22 is to down-modulate the B cell activation threshold. It can also mediate cell binding through binding to cells containing appropriate sialoglycoconjugates.
CD22 is expressed in most B-cell leukemias and lymphomas including acute lymphoblastic leukemia (B-ALL), chronic lymphocytic leukemia (B-CLL) and especially acute non-lymphocytic leukemia (ANLL).
For the treatment or diagnosis of such CD22 expression-related diseases, CD22-specific antibodies have been developed. International Patent Publication No. WO1998-041641 discloses a recombinant anti-CD22 antibody having cysteine residues at positions $V_H$ 44 and $V_L$ 100, and International Patent Publication No. WO 1998-042378 discloses a CD22 antibody for the treatment of B-cell malignancies.

[Disclosure]

[Technical Problem]

In the present invention, in order to develop an antibody that more specifically binds to CD22, five novel antibodies (1C2, 9E9, 2A9, 2G1 and 6B3) were established by screening an antibody that binds to CD22 and it was confirmed that the 5 selected in the present invention specifically bound to CD22 antigen. In addition, using the CD22-specific antibody of the present invention, a chimeric antigen receptor targeting CD22 and CAR-T cells were prepared, and it was confirmed that CD22-CAR-T cells not only bound to CD22, but also effectively killed CD22-expressing cells (B-cell lymphoma).
Accordingly, it is an object of the present invention to provide an antibody that specifically binds to CD22.
Another object of the present invention is to provide a polynucleotide encoding the antibody, a vector expressing the antibody, and a recombinant cell transformed with the vector.
Another object of the present invention is a chimeric antigen receptor comprising the antibody, a polynucleotide encoding the chimeric antigen receptor targeting CD22, a vector comprising the same, and an immune effector expressing the chimeric antigen receptor comprising the polynucleotide or the vector.
Another object of the present invention is to provide a pharmaceutical composition for preventing or treating diseases mediated by CD22-expressing cells, including the present antibody or the immune effector cells expressing chimeric antigen receptor targeting CD22.
Another object of the present invention is to provide a composition for diagnosing or monitoring a disease mediated by a cell expressing CD22 comprising the antibody.

[Technical solution]

In order to achieve the above object,
The present invention provides an antibody specifically binding to CD22 or a fragment thereof, comprising:

(a) a heavy chain variable region comprising a CDR1 region represented by the amino acid of SEQ ID NO: 1, a CDR2 region represented by the amino acid of SEQ ID NO: 2 and a CDR3 region represented by the amino acid of SEQID NO: 3, and a light chain variable region comprising a CDR1 region represented by the amino acid of SEQ ID NO: 4, a CDR2 region represented by the amino acid of SEQ ID NO: 5, and a CDR3 region represented by the amino acid sequence represented by SEQ ID NO: 6;

(b) a heavy chain variable region comprising a CDR1 region represented by the amino acid of SEQ ID NO: 11, a CDR2 region represented by the amino acid of SEQ ID NO: 12 and a CDR3 region represented by the amino acid of SEQ ID NO: 13 and a light chain variable region comprising a CDR1 region represented by the amino acid of SEQ ID NO: 14, a CDR2 region represented by amino acids of SEQ ID NO: 15 and a CDR3 region represented by amino acids of SEQ ID NO: 16;

(c) a heavy chain variable region comprising a CDR1 region represented by the amino acid of SEQ ID NO: 21, a CDR2 region represented by the amino acid of SEQ ID NO: 22 and a CDR3 region represented by the amino acid of SEQ ID NO: 23 and a light chain variable region comprising a CDR1 region represented by the amino acid of SEQ ID NO: 24, a CDR2 region represented by the amino acid of SEQ ID NO: 25 and a CDR3 region represented by the amino acid of SEQ ID NO: 26;

(d) a heavy chain variable region comprising a CDR1 region represented by the amino acid of SEQ ID NO: 31, a CDR2 region represented by the amino acid of SEQ ID NO: 32 and a CDR3 region represented by the amino acid of SEQ ID NO: 33 and a light chain variable region comprising a CDR1 region represented by the amino acid of SEQ ID NO: 34, a CDR2 region represented by the amino acid of SEQ ID NO: 35 and a CDR3 region represented by the amino acid of SEQ ID NO: 36; or

(e) a heavy chain variable region comprising a CDR1 region represented by the amino acid of SEQ ID NO: 41, a CDR2 region represented by the amino acid of SEQ ID NO: 42 and a CDR3 region represented by the amino acid of SEQ ID NO: 43 and a light chain variable region comprising a CDR1 region represented by the amino acid of SEQ ID NO: 44, a CDR2 region represented by the amino acid of SEQ ID NO: 45 and a CDR3 region represented by the amino acid of SEQ ID NO: 46.

In a preferred embodiment of the present invention, the antibody may be a monoclonal antibody, preferably a single-chain variable fragment (scFv).

In another preferred embodiment of the present invention,

the antibody (a) has a heavy chain variable region represented by the amino acid of SEQ ID NO: 7 and a light chain variable region represented by the amino acid of SEQ ID NO: 8,

the (b) antibody has a heavy chain variable region represented by the amino acid of SEQ ID NO: 17 and a light chain variable region represented by the amino acid of SEQ ID NO: 18,

the (c) antibody has a heavy chain variable region represented by the amino acid of SEQ ID NO: 27 and a light chain variable region represented by the amino acid of SEQ ID NO: 28,

the (d) antibody has a heavy chain variable region represented by the amino acid of SEQ ID NO: 37 and a light chain variable region represented by the amino acid of SEQ ID NO: 38, or

the (e) antibody has a heavy chain variable region represented by the amino acid of SEQ ID NO: 47 and a light chain variable region represented by the amino acid of SEQ ID NO: 48.

To achieve another object, the present invention provides a polynucleotide encoding the antibody that specifically binds to CD22.

In addition, the present invention provides a vector comprising a polynucleotide encoding the antibody that specifically binds to CD22.

In addition, the present invention provides a recombinant cell that produces an antibody or fragment thereof that specifically binds to CD22 transformed with the vector.

To achieve another object, the present invention provides a chimeric antigen receptor (CAR) comprising: a CD22-binding domain; a transmembrane domain; a costimulatory domain; and an intracellular signal transduction domain, wherein the CD22-binding domain may be selected from an antibody or fragment thereof capable of specifically binding to CD22 of the present invention.

In a preferred embodiment of the present invention, the transmembrane domain may be derived from a protein selected from the group consisting of CD8α, CD4, CD28, CD137, CD80, CD86, CD152 and PD1.

In another preferred embodiment of the present invention, the costimulatory domain may be derived from a protein selected from the group consisting of CD28, 4-1BB, OX-40 and ICOS, and the signaling domain may be derived from CD3ζ.

In another preferred embodiment of the present invention, it may further include a hinge region located between the C-terminus of the CD22-binding domain and the N-terminus of the transmembrane domain, the hinge region may be derived from CD8α.

To achieve another object, the present invention provides a polynucleotide encoding the chimeric antigen receptor (CAR).

The present invention also provides a vector comprising a polynucleotide encoding a chimeric antigen receptor (CAR).

In a preferred embodiment of the present invention, the vector may be a plasmid, a retroviral vector or a lentiviral vector.

In addition, the present invention provides an immune effector cell expressing the chimeric antigen receptor (CAR) comprising the polynucleotide encoding the chimeric antigen receptor (CAR).

In a preferred embodiment of the present invention, the immune effector cells may be T cells.

To achieve another object, the present invention provides a pharmaceutical composition for use in preventing or treating a disease mediated by cells expressing CD22, comprising the antibody specifically binding to CD22 or the fragment thereof of the invention or the immune effector cell expressing a chimeric antigen receptor targeting CD22 of the invention.

In a preferred embodiment of the present invention, the disease mediated by cells expressing CD22 is lymphoma, non-Hogkins lymphoma (NHL), aggressive NHL, relapsed aggressive NHL, relapsed delayed NHL, refractory Sexual NHL, refractory delayed NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma.

In another preferred embodiment of the present invention, the composition may include a therapeutic agent for a disease mediated by cells expressing CD22.

In addition, the present invention provides a composition for diagnosing or monitoring a disease mediated by cells expressing CD22, including the present antibody that specifically binds to CD22.

[Advantageous Effects]

In the present invention, five novel antibodies (1C2, 9E9, 2A9, 2G1 and 6B3) were established by screening antibodies that more specifically bind to CD22, and it was confirmed that the novel antibodies specifically bound to the CD22 antigen.

In addition, it was confirmed that the chimeric antigen receptor (CAR) and CAR-T cells targeting CD22 prepared using the established antibody not only effectively bound to CD22, but also activated the CAR-T cells bound to CD22.

Furthermore, since it was confirmed that the CAR-T cells of the present invention effectively killed CD22-expressing cells, the CD22-specific antibody, the CD22-specific antibody of the present invention, the chimeric antigen receptor prepared using the same, and CAR-T cells of the present invention can be applied to the prevention or treatment of a disease mediated by cells expressing CD22.

[Description of Drawings]

Figure 1 is data confirming the binding affinity to CD22 of 1C2, 9E9, 2A9, 2G1 and 6B3 antibodies selected in the present invention by FACS.

Figure 2 is a schematic diagram showing a lentiviral vector expressing a chimeric antigen receptor (CD22-CAR) targeting CD22 and a chimeric antigen receptor expressed in T cells.

Figure 3 is a schematic diagram illustrating a method for preparing CD22-CAR-T cells using a lentivirus expressing CD22-CAR.

Figure 4 is data confirming the CD22 binding ability of CD22-CAR-T cells prepared using each of 1C2, 9E9, 2G1 and 6B3 antibodies.

Figure 5 is data confirming the level of IFNy expression by CD22-CAR-T cells in the presence of target cells to confirm the activation of CD22-CAR-T cells prepared using each of 1C2, 9E9, 2G1 and 6B3 antibodies.

Figure 6 is data confirming the killing effect of target cells by CD22-CAR-T cells prepared using each of 1C2, 9E9, 2G1 and 6B3 antibodies.

[Mode of the Invention]

Hereinafter, the present invention will be described in detail.

**Antibodies that specifically bind to CD22**

In one aspect, the present invention provides an antibody or fragment thereof that specifically binds to CD22, comprising:

(a) a heavy chain variable region comprising a CDR1 region represented by the amino acid of SEQ ID NO: 1, a CDR2 region represented by the amino acid of SEQ ID NO: 2 and a CDR3 region represented by the amino acid of SEQ ID NO: 3, and a light chain variable region comprising a CDR1 region represented by the amino acid of SEQ ID NO: 4, a CDR2 region represented by the amino acid of SEQ ID NO: 5, and a CDR3 region represented by the amino acid sequence represented by SEQ ID NO: 6;

(b) a heavy chain variable region comprising a CDR1 region represented by the amino acid of SEQ ID NO: 11, a CDR2 region represented by the amino acid of SEQ ID NO: 12 and a CDR3 region represented by the amino acid of SEQ ID NO: 13 and a light chain variable region comprising a CDR1 region represented by the amino acid of SEQ ID NO: 14, a CDR2 region represented by amino acids of SEQ ID NO: 15 and a CDR3 region represented by amino acids of SEQ ID NO: 16;

(c) a heavy chain variable region comprising a CDR1 region represented by the amino acid of SEQ ID NO: 21, a CDR2 region represented by the amino acid of SEQ ID NO: 22 and a CDR3 region represented by the amino acid of SEQ ID NO: 23 and a light chain variable region comprising a CDR1 region represented by the amino acid of SEQ ID NO: 24, a CDR2 region represented by the amino acid of SEQ ID NO: 25 and a CDR3 region represented by the amino acid of SEQ ID NO: 26;

(d) a heavy chain variable region comprising a CDR1 region represented by the amino acid of SEQ ID NO: 31, a CDR2 region represented by the amino acid of SEQ ID NO: 32 and a CDR3 region represented by the amino acid of SEQ ID NO: 33 and a light chain variable region comprising a CDR1 region represented by the amino acid of SEQ ID NO: 34, a CDR2 region represented by the amino acid of SEQ ID NO: 35 and a CDR3 region represented by the amino acid of SEQ ID NO: 36; or

(e) a heavy chain variable region comprising a CDR1 region represented by the amino acid of SEQ ID NO: 41, a CDR2 region represented by the amino acid of SEQ ID NO: 42 and a CDR3 region represented by the amino acid of SEQ ID NO: 43 and a light chain variable region comprising a CDR1 region represented by the amino acid of SEQ ID NO: 44, a CDR2 region represented by the amino acid of SEQ ID NO: 45 and a CDR3 region represented by the amino acid of SEQ ID NO: 46.

In the present invention, the antibody may be a monoclonal antibody. In the present invention, the term "monoclonal antibody" is an antibody produced by a single antibody-forming cell, and has a uniform primary structure (amino acid sequence). It recognizes only one antigenic determinant and is generally produced by culturing a hybridoma cell in which cancer cells and antibody-producing cells are fused. can also be produced. In addition, the antibody may be used by humanizing the remaining parts except for a CDR parts, if necessary.

As used herein, the term "CDR", ie, "complementarity determining region", refers to a non-contiguous antigen binding site found within the variable region of both heavy and light chain polypeptides.

As used herein, the term "humanized antibody" is an antibody that possesses an amino acid sequence corresponding to that of an antibody produced by a human and/or is made using one of the techniques for making human antibodies as disclosed herein. This definition of a humanized antibody specifically excludes a humanized antibody comprising non-human antigen-binding residues.

In the present invention, the term "antibody" can be used not only in a complete form having two full-length light chains and two full-length heavy chains, but also fragments of antibody molecule. A fragment of antibody molecule means a fragment having at least a peptide tag (epitope) binding function, and includes scFv, Fab, F(ab'), F(ab')$_2$, a single domain, etc.

Among antibody fragments, Fab has a structure having variable regions of light and heavy chains, a constant region of light chain and the first constant region of heavy chain (CH1), and has one antigen-binding site. Fab' differs from Fab in that it has a hinge region comprising one or more cysteine residues at the C terminus of the heavy chain CH1 domain. F(ab')$_2$ antibody is produced by forming a disulfide bond with a cysteine residue in the hinge region of Fab'. Fv is a minimal antibody fragment having only a heavy chain variable region and a heavy chain variable region. Recombinant technology for generating an Fv fragment is described in International Patent Publications WO 88/10649, WO 88/106630, WO 88/07085, WO 88 /07086 and WO 88/09344. A double chain Fv (dsFv) has a disulfide bond, and a heavy chain variable region and a heavy chain variable region are connected, and a single chain Fv (scFv) is generally connected through a peptide linker, the variable region of the heavy chain and the variable region of the light chain are covalently bonded. Such an antibody fragment can be obtained using a proteolytic enzyme (for example, Fab can be obtained by restriction digestion of the entire antibody with papain, and F(ab')$_2$ fragment can be obtained by digestion with pepsin). Preferably, it can be produced through genetic recombination technology.

The monoclonal antibody that specifically binds to CD22 of the present invention can be prepared by using all or part of the CD22 protein as an immunogen (or antigen). More specifically, as an immunogen, CD22, a fusion protein containing CD22 protein, or a carrier containing CD22 protein, if necessary, together with an adjuvant (e.g., Freund adjuvant), is injected once or more by subcutaneous, intramuscular, intravenous, intraperitoneal in mammals except for humans to achieve an immunization. The mammals other than humans are preferably mice, rats, hamsters, malmots, chickens, rabbits, cats, dogs, pigs, goats, sheep, donkeys, horses or cattle (including transgenic animals engineered to produce an antibody from other animals such as mice to produce human antibody), more preferably mouse, rat, hamster, malmot, chicken or rabbit. Antibody-producing cells can be obtained from the immune-sensitized mammal about 1 to 10 days after the final immunization by performing immunization 1 to 4 times every 1 to 21 days from the first immunization. The number of times and intervals for immunization can be appropriately changed depending on the characteristics of the immunogen to be used.

Preparation of a hybridoma secreting a monoclonal antibody can be carried out according to the method of Keira and Mirstein et al. (Nature, 1975, Vol. 256, p. 495-497) and a method similar thereto. Hybridomas can be produced by cell fusion of mammal-derived myeloma cells without autologous antibody-producing ability and antibody-producing cells contained in the group consisting of spleen, lymph node, bone marrow and tonsils, preferably spleen. The mammal may be a mouse, rat, malmot, hamster, chicken, rabbit or human, preferably a mouse, rat, chicken or human.

For cell fusion, for example, a fusion promoter including polyethylene glycol or Sendai virus or a method by electric pulse is used, for example, in a fusion medium containing a fusion promoter, antibody-producing cells and mammalian-derived cells capable of indefinite proliferation. Cells are suspended at a ratio of about 1:1 to 1:10, and in this state, cultured at about 30 to 40°C for about 1 to 5 minutes. As the fusion medium, for example, MEM medium, RPMI1640 medium, and Iscove's Modified Dulbecco's Medium may be used, and it is preferable to exclude sera such as bovine serum.

In the method of screening the hybridoma clones producing the monoclonal antibody, first, the fusion cells obtained as described above are transferred to a selection medium such as HAT medium, and cultured at about 30 to 40°C. for about 3 days to 3 weeks to kill cells other than hybridomas. Then, after culturing the hybridoma on a microtiter plate, etc., the part with increased reactivity between the immunogen used for the immune response of animals other than humans described above and the culture supernatant was subjected to RIA (radioactive substance-marked immuno antibody) or ELISA (Enzyme-Linked Immunosorbent Assay). The clone producing the monoclonal antibody found above shows specific binding ability to the immunogen.

The monoclonal antibody of the present invention can be obtained by culturing such a hybridoma in vitro or in vivo. For culturing, a conventional method for culturing cells derived from mammals is used, and for collecting monoclonal antibody from a culture or the like, a conventional method in this field for purifying an antibody in general is used. As each method, for example, salting out, dialysis, filtration, concentration, centrifugation, fractional precipitation, gel filtration chromatography, ion exchange chromatography, affinity chromatography, high-performance liquid chromatography, gel electrophoresis or isoelectric point electrophoresis, etc. can be applied, and these are applied in combination as needed. The purified monoclonal antibody is then concentrated and dried to be in a liquid or solid state depending on the use.

In addition, the monoclonal antibody of the present invention comprises DNAs encoding heavy chain and light chain variable regions, respectively, with known DNA encoding heavy chain and light chain constant regions (e.g., Japan 2007-252372). No. publication) and each ligated gene is synthesized by PCR method or chemical synthesis, and the transformant is obtained by transplanting a known expression vector (pcDNA 3.1 (sold by Invitrogen), etc.) that enables expression of the gene. It can be obtained by preparing an antibody and expressing it in a host such as CHO cells or Escherichia coli, and purifying the antibody from such a culture medium using a protein A or G (Protein A or G) column or the like.

In a specific embodiment of the present invention, in order to prepare an antibody that specifically binds to CD22, hybridomas producing an anti-CD22 antibody were prepared and screened, and 5 kinds of antibodies (scFv) that specifically binds to CD22 (scFv) were selected and designated as 1C2, 9E9, 2A9, 2G1 and 6B3, respectively.

(a) It was confirmed that the 1C2 antibody had a heavy chain variable region including a CDR1 region represented by the amino acid of SEQ ID NO: 1 (GYTFTSYWMNW), a CDR2 region represented by the amino acid of SEQ ID NO: 2 (IDPSDSET) and a CDR3 region represented by the amino acid of SEQ ID NO: 3 (ARWGNYDYDVWAMDY), and a light chain variable region including a CDR1 region represented by the amino acid of SEQ ID NO: 4 (QNIVHLNGNTF), a CDR2 region represented by the amino acid of SEQ ID NO: 5 (KVS) and a CDR3 region represented by the amino acid of SEQ ID NO: 6 (FQGSHVPYT).

In addition, it was confirmed that 1C2 antibody contained a heavy chain variable region represented by the amino acid of SEQ ID NO: 7 and a light chain variable region represented by the amino acid of SEQ ID NO: 8, and the heavy chain variable region was encoded by the nucleotide sequence of SEQ ID NO: 9, and the light chain variable region was encoded by the nucleotide sequence of SEQ ID NO: 10.

(b) It was confirmed that 9E9 antibody had a heavy chain variable region including a CDR1 region represented by the amino acid of SEQ ID NO: 11 (GYAFSIYW), a CDR2 region represented by the amino acid of SEQ ID NO: 12 (IYPGDGDT) and a CDR3 region represented by the amino acid of SEQ ID NO: 13 (ARRGITPWFAY), and a light chain variable region including a CDR1 region represented by the amino acid of SEQ ID NO: 14 (QSLLNSSNQKNY), a CDR2 region represented by the amino acid of SEQ ID NO: 15 (FAS) and a CDR3 region represented by the amino acid of SEQ ID NO: 16 (CQQHYSTPLT).

In addition, it was confirmed that 9E9 antibody contained a heavy chain variable region represented by the amino acid of SEQ ID NO: 17 and a light chain variable region represented by the amino acid of SEQ ID NO: 18, and the heavy chain variable region was encoded by the nucleotide sequence of SEQ ID NO: 19, and the light chain variable region was encoded by the nucleotide sequence of SEQ ID NO: 20.

(c) It was confirmed that 2A9 antibody had a heavy chain variable region including a CDR1 region represented by the amino acid of SEQ ID NO: 21 (GYTFTTYP), a CDR2 region represented by the amino acid of SEQ ID NO: 22 (FHPYNDDT) and a CDR3 region represented by the amino acid of SEQ ID NO: 23 (ARGDYYVSSWYFDV), and a light chain variable region including a CDR1 region represented by the amino acid of SEQ ID NO: 24 (QNVGTN), a CDR2 region (SAS) represented by the amino acid of SEQ ID NO: 25 and a CDR3 region represented by the amino acid of SEQ ID NO: 26 (QQYNSYPLT).

In addition, it was confirmed that 2A9 antibody contained a heavy chain variable region represented by the amino acid of SEQ ID NO: 27 and a light chain variable region represented by the amino acid of SEQ ID NO: 28, and the heavy chain variable region was encoded by the nucleotide sequence of SEQ ID NO: 29, and the light chain variable region was encoded by the nucleotide sequence of SEQ ID NO: 30.

(d) It was confirmed that the 2G1 antibody had a heavy chain variable region including a CDR1 region represented by the amino acid of SEQ ID NO: 31 (GFSLTSYDI), a CDR2 region represented by the amino acid of SEQ ID NO: 32 (IWTGGGT) and a CDR3 region represented by the amino acid of SEQ ID NO: 33 (VPHYYGYAMDYW), and a light chain variable region including a CDR1 region represented by the amino acid of SEQ ID NO: 34 (QDINKY), a CDR2 region represented by the amino acid of SEQ ID NO: 35 (YTS) and a CDR3 region represented by the amino acid of SEQ ID NO: 36 (LQYDNLLT).

In addition, it was confirmed that 2G1 antibody contained a heavy chain variable region represented by the amino acid of SEQ ID NO: 37 and a light chain variable region represented by the amino acid of SEQ ID NO: 38, and the heavy chain variable region was encoded by the nucleotide sequence of SEQ ID NO: 39, and the light chain variable region was encoded by the nucleotide sequence of SEQ ID NO: 40.

(e) It was confirmed that the 6B3 antibody had a heavy chain variable region including a CDR1 region represented by the amino acid of SEQ ID NO: 41 (GYTFTDYN), a CDR2 region represented by the amino acid of SEQ ID NO: 42 (IYPYNGGT) and a CDR3 region represented by the amino acid of SEQ ID NO: 43 (ARLYYYGRTSYWG), and a light chain variable region including a CDR1 region represented by the amino acid of SEQ ID NO: 44 (QNVGTY), a CDR2 region (SAS) represented by the amino acid of SEQ ID NO: 45 and a CDR3 region represented by the amino acid of SEQ ID NO: 46 (HQYDSYPYT).

In addition, it was confirmed that 6B3 antibody contained a heavy chain variable region represented by the amino acid of SEQ ID NO: 47 and a light chain variable region represented by the amino acid of SEQ ID NO: 48, and the heavy chain variable region was encoded by the nucleotide sequence of SEQ ID NO: 49, and the light chain variable region was encoded by the nucleotide sequence of SEQ ID NO: 50.

The CD22-specific antibody of the present invention may be preferably an scFv (single chain variable fragment), and can be produced through genetic recombination technology so that the heavy chain variable region and the light chain variable region can be linked with a linker. The linker may be preferably represented by the amino acid sequence of SEQ ID NO: 51 or the nucleotide sequence of SEQ ID NO: 52, but is not limited thereto.

When linked by a light chain variable region-linker-heavy chain variable region, the 1C2 antibody may be the amino acid sequence of SEQ ID NO: 53 or the nucleotide sequence of SEQ ID NO: 54, the 9E9 antibody may be the amino acid sequence of SEQ ID NO: 55 or the nucleotide sequence of SEQ ID NO: 56, the 2A9 antibody may be the amino acid sequence of SEQ ID NO: 57 or the nucleotide sequence of SEQ ID NO: 58, the 2G1 antibody may be the amino acid sequence of SEQ ID NO: 59 or the nucleotide sequence of SEQ ID NO: 60, and the 6B3 antibody may be the amino acid sequence of SEQ ID NO: 61 or the nucleotide sequence of SEQ ID NO: 62.

In another aspect, the present invention relates to a polynucleotide encoding the antibody that specifically binds to CD22.

As used herein, the term "polynucleotide" generally refers to a nucleic acid molecule, deoxyribonucleotide or ribonucleotide, or an analog thereof, separated by any length. In some embodiments, a polynucleotide of the present invention can be prepared by (1) in-vitro amplification, such as polymerase chain reaction (PCR) amplification; (2) cloning and recombination; (3) purification such as digestion and gel electrophoretic separation; (4) synthesis such as chemical synthesis, and preferably, the isolated polynucleotide is prepared by recombinant DNA technology. In the present invention, the nucleic acid for encoding the antibody or antigen-binding fragment thereof can be prepared by various methods known in the art, including, but not limited to, restriction fragment operation of synthetic oligonucleotides or application of SOE PCR.

In another aspect, the present invention relates to a vector comprising a polynucleotide encoding an antibody that specifically binds to CD22, and a recombinant cell transformed with the vector.

In the present invention, the term "vector (expression vector)" refers to a gene preparation including essential regulatory elements such as a promoter so that a target gene can be expressed in an appropriate host cell. A vector may be selected from one or more of a plasmid, a retroviral vector, and a lentiviral vector. Upon transformation into an appropriate host, a vector can replicate and function independently of the host genome, or in some cases can be integrated into the genome itself.

In addition, a vector may contain expression control elements that allow the coding region to be accurately expressed in a suitable host. Such regulatory elements are well known to those skilled in the art and include, for example, promoters, ribosome-binding sites, enhancers and other regulatory elements for regulating gene transcription or mRNA translation. The specific structure of the expression control sequence may vary depending on the function of the species or cell type, but generally contains 5' non-translated sequence, and a 5' or 3' non-translated sequence participating in transcription initiation and translation initiation, respectively, such as TATA box, capped sequence, CAAT sequence, etc. For example, a 5' non-transcriptional expression control sequence can include a promoter region that can include a promoter sequence for transcription and control of a functionally linked nucleic acid.

As used herein, the term "promoter" means a minimal sequence sufficient to direct transcription. In addition, promoter constructs sufficient to allow expression of a regulatable promoter-dependent gene induced by cell type-specific or external signals or agents may be included, and these constructs may be located in the 5' or 3' portion of the gene. Both conservative and inducible promoters are included. Promoter sequences may be derived from prokaryotes, eukaryotes or viruses.

In the present invention, the term "transformant" refers to a cell transformed by introducing a vector having a polynucleotide encoding one or more target proteins into a host cell, and a method for introducing the expression a vector into the host cell to form a transformant are such as a calcium phosphate method or a calcium chloride/rubidium chloride method, an electroporation method, an electroinjection method, a chemical treatment method such as PE.G., a method using a gene gun, and the like (Sambrook, J., et al., Molecular Cloning, A Laboratory Manual(2nd ed.), Cold Spring Harbor Laboratory, 1. 74, 1989).

When the transformant expressing the vector is cultured in a nutrient medium, an antibody protein can be produced and isolated in large quantities. Medium and culture conditions can be appropriately selected and used depending on the host cell. During culture, conditions such as temperature, medium pH, and culture time should be appropriately adjusted to be suitable for cell growth and mass production of proteins.

The vector according to the present invention can be transformed into a host cell, preferably a mammalian cell, for the production of the antibody. Suitable host cells capable of expressing fully glycosylated proteins include COS-1 (e.g. ATCC CRL 1650), COS-7 (e.g. ATCC CRL-1651), HEK293, BHK21 (e.g. ATCC CRL-10), CHO (e.g. ATCC CRL 1610) and BSC-1 (e.g. ATCC CRL-26) cell lines, Cos-7 cells, CHO cells, hep G2 cells, P3X63Ag8.653, SP2/0 -Agl4, 293 cells, HeLa cells, etc., and these cells are readily available from, for example, ATCC (American Type Culture Collection, USA).

## Chimeric antigen receptor targeting CD22

The present invention also relates to a chimeric antigen receptor (CAR) comprising: a CD22-binding domain; a transmembrane domain; a costimulatory domain; and an intracellular signal transduction domain,
wherein the CD22-binding domain is an antibody specifically binding to CD22 or a fragment thereof, comprising:

   (a) a heavy chain variable region comprising a CDR1 region represented by the amino acid of SEQ ID NO: 1, a CDR2 region represented by the amino acid of SEQ ID NO: 2 and a CDR3 region represented by the amino acid of SEQ ID NO: 3, and a light chain variable region comprising a CDR1 region represented by the amino acid of SEQ ID NO: 4, a CDR2 region represented by the amino acid of SEQ ID NO: 5, and a CDR3 region represented by the amino acid sequence represented by SEQ ID NO: 6;
   (b) a heavy chain variable region comprising a CDR1 region represented by the amino acid of SEQ ID NO: 11, a CDR2 region represented by the amino acid of SEQ ID NO: 12 and a CDR3 region represented by the amino acid of SEQ ID NO: 13 and a light chain variable region comprising a CDR1 region represented by the amino acid of SEQ ID NO: 14, a CDR2 region represented by amino acids of SEQ ID NO: 15 and a CDR3 region represented by amino acids of SEQ ID NO: 16;
   (c) a heavy chain variable region comprising a CDR1 region represented by the amino acid of SEQ ID NO: 21, a CDR2 region represented by the amino acid of SEQ ID NO: 22 and a CDR3 region represented by the amino acid of SEQ ID NO: 23 and a light chain variable region comprising a CDR1 region represented by the amino acid of SEQ ID NO: 24, a CDR2 region represented by the amino acid of SEQ ID NO: 25 and a CDR3 region represented by the amino acid of SEQ ID NO: 26;

(d) a heavy chain variable region comprising a CDR1 region represented by the amino acid of SEQ ID NO: 31, a CDR2 region represented by the amino acid of SEQ ID NO: 32 and a CDR3 region represented by the amino acid of SEQ ID NO: 33 and a light chain variable region comprising a CDR1 region represented by the amino acid of SEQ ID NO: 34, a CDR2 region represented by the amino acid of SEQ ID NO: 35 and a CDR3 region represented by the amino acid of SEQ ID NO: 36; or

(e) a heavy chain variable region comprising a CDR1 region represented by the amino acid of SEQ ID NO: 41, a CDR2 region represented by the amino acid of SEQ ID NO: 42 and a CDR3 region represented by the amino acid of SEQ ID NO: 43 and a light chain variable region comprising a CDR1 region represented by the amino acid of SEQ ID NO: 44, a CDR2 region represented by the amino acid of SEQ ID NO: 45 and a CDR3 region represented by the amino acid of SEQ ID NO: 46.

As used herein, the term "chimeric antigen receptor (CAR)" generally refers to a fusion protein containing an extracellular domain having the ability to bind an antigen and one or more intracellular domains. A CAR is a core part of a chimeric antigen receptor T cell (CAR-T) and may contain an antigen binding domain, a transmembrane domain, a co-stimulatory domain, and an intracellular signal transduction domain. A CAR can be combined with a T cell receptor-activating intracellular domain based on the antigen (e.g., CD22) specificity of the antibody. Genetically modified CAR-expressing T cells can specifically identify and eliminate target antigen-expressing malignant cells.

In the present invention, the term "CD22-binding domain" generally refers to a domain capable of specifically binding to a CD22 protein. For example, the CD22-binding domain may contain an anti-CD22 antibody or a fragment thereof capable of specifically binding to a human CD22 polypeptide or a fragment thereof expressed in B cells.

In the present invention, the term "binding domain " can be used interchangeably refers to "extracellular domain", "extracellular binding domain", "antigen-specific binding domain" and "extracellular antigen-specific binding domain" and refers to a CAR domain or fragment that has the ability to specifically bind to a target antigen (e.g., CD22).

In the present invention, anti-CD22 antibody or fragment thereof is the above-described anti-CD22 antibody, a monoclonal antibody, preferably a single chain variable fragment (scFv). Specifically, it can be prepared using 1C2, 9E9, 2A9, 2G1 or 6B3 antibodies specific for CD22 of the present invention, and 1C2, 9E9, 2G1 or 6B3 antibodies are preferably used in the present invention.

In the present invention, a signal peptide may be further included at the N-terminus of the CD22-binding domain, and the "signal peptide" generally refers to a peptide chain for guiding protein transduction. The signal peptide may be a short peptide having a length of 5 to 30 amino acids, preferably represented by the amino acid sequence of SEQ ID NO: 70.

In the present invention, it may further include a hinge region located between the C terminus of the CD22-binding domain and the N terminus of a transmembrane domain, wherein the hinge region is derived from CD8α, and preferably, it can be represented by the amino acid sequence of SEQ ID NO: 71. The "hinge region" generally refers to the linking region between an antigen-binding region and an immune cell Fc receptor (FcR)-binding region.

In the present invention, "a transmembrane domain" refers to a domain of a CAR that generally passes through a cell membrane and is connected to an intracellular signal transduction domain to play a role in signal transduction. The transmembrane domain may be derived from a protein selected from the group consisting of CD8α, CD4, CD28, CD137, CD80, CD86, CD152 and PD1, and preferably may be represented by the amino acid sequence of SEQ ID NO: 72.

In the present invention, "costimulatory domain" generally refers to an intracellular domain capable of providing immune-stimulatory molecules, which are cell surface molecules necessary for an effective response of lymphocytes to antigens. The costimulatory domain described above may comprise a costimulatory domain of CD28, and may comprise a costimulatory domain of the TNF receptor family, such as the costimulatory domain of OX40 and 4-1BB, preferably it may be 4-1BB represented by the amino acid sequence of SEQ ID NO: 73.

In the present invention, "intracellular signal transduction domain" generally refers to a domain located inside a cell and capable of transmitting a signal. In the present invention, the intracellular signal transduction domain is an intracellular signal transduction domain of the chimeric antigen receptor. For example, the intracellular signal transduction domain may be selected from CD3ζ intracellular domain, CD28 intracellular domain, CD28 intracellular domain, 4-1BB intracellular domain and OX40 intracellular domain, and preferably it may be CD3ζ represented by the amino acid sequence of SEQ ID NO: 74.

## Chimeric Antigen Receptor Encoding Polynucleotides and Chimeric Antigen Receptor Expression Vectors

In another aspect, the present invention relates to a polynucleotide encoding the chimeric antigen receptor (CAR).

In the present invention, the polynucleotide encoding the chimeric antigen receptor (CAR) comprises a polynucleotide encoding a CD22-binding domain; a polynucleotide encoding a transmembrane domain; a polynucleotide encoding a co-stimulatory domain; and a polynucleotide encoding an intracellular signaling domain.

The polynucleotide encoding the CD22-binding domain may be a polynucleotide encoding the antibody specific for CD22 of the present invention, preferably 1C2, 9E9, 2G1 or 6B3 antibody, and a light chain variable region and a heavy chain variable region may be in the form of scFv linked by a linker, and the specific nucleotide sequence thereof may be the same as described above.

Preferably, the polynucleotide encoding the chimeric antigen receptor (CAR) of the present invention may comprise:

a signal peptide represented by the nucleotide sequence of SEQ ID NO: 64;

1C2 antibody represented by the nucleotide sequence of SEQ ID NO: 54, 9E9 antibody represented by the nucleotide sequence of SEQ ID NO: 56, 2G1 antibody represented by the nucleotide sequence of SEQ ID NO: 60, or 6B3 antibody represented by the nucleotide sequence of SEQ ID NO: 62;

a transmembrane domain represented by the nucleotide sequence of SEQ ID NO: 66;

4-1BB (costimulatory domain) represented by the nucleotide sequence of SEQ ID NO: 67; and

an intracellular signal transduction domain (CD3ζ) represented by the nucleotide sequence of SEQ ID NO: 68.

In addition, between the polynucleotide encoding the CD22-binding domain and the transmembrane domain, a polynucleotide encoding a hinge region may be further included. Preferably CD8 hinge region represented by the nucleotide sequence of SEQ ID NO: 65 can be further included.

In another aspect, the present invention relates to a vector comprising a polynucleotide encoding the chimeric antigen receptor (CAR).

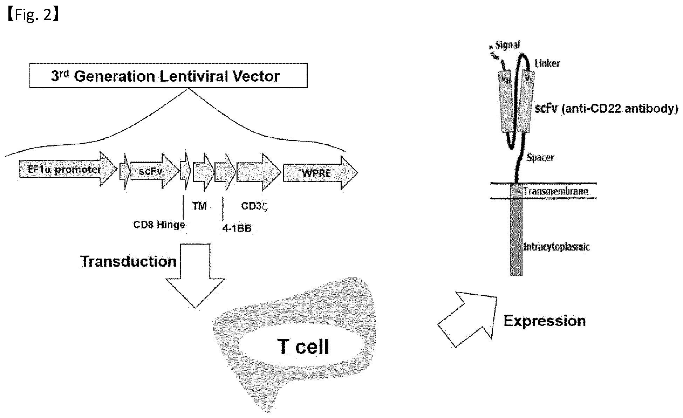

In a specific embodiment of the present invention, the vector is a recombinant virus a vector, preferably a lentivirus vector, and comprises an operably linked EF1α promoter; a polynucleotide encoding a signal peptide; a polynucleotide encoding a CD22-binding domain; a polynucleotide encoding a transmembrane domain; and a polynucleotide encoding an intracellular signal transduction domain, and may further include a woodchuck hepatitis virus post-transcriptional regulatory element (WPRE) to increase protein expression (refer to FIG. 2).

The EF1α promoter may be represented by the nucleotide sequence of SEQ ID NO: 63, and if necessary, has 90% or more, 93% or more, 95% or more, 96% or more, 97% or more, 98% or more, or 99% or more identical sequences of the nucleotide sequence of SEQ ID NO: 63.

In addition, the promoter is operably linked to induce expression of an anti-CD22 antibody (scFv), which is a CD22-binding domain.

Biological methods for introducing polynucleotides into host cells include the use of DNA and RNA vectors. Viral vectors, and in particular retroviral vectors, have become the most widely used methods for inserting genes into mammalian, e.g., human cells. Other virus vector may be derived from lentiviruses, poxviruses, herpes simplex viruses, adenoviruses and adeno-associated viruses, and the like.

Chemical means for introducing polynucleotides into host cells include colloidal dispersion systems such as macromolecular complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle).

When a non-viral delivery system is used, an exemplary delivery vehicle is a liposome. The use of lipid preparations is contemplated for the introduction of nucleic acids into host cells (in vitro, ex vivo or in vivo). In another aspect, the nucleic acid may be associated with a lipid. Nucleic acids associated with lipids may be encapsulated within the aqueous interior of the liposome, interspersed within the lipid bilayer of the liposome, attached to the liposome via a linking molecule associated with both the liposome and oligonucleotide, captured within the liposome, complexed with the liposome, dispersed in a lipid-containing solution, mixed with a lipid or combined with a lipid, contained as a suspension in a lipid, contained or complexed with micelles, or otherwise associated with a lipid. Lipid, lipid/DNA or lipid/expression a vector association composition is not limited to any particular structure in solution.

**Chimeric Antigen Receptor (CAR) Expression Immune Effector Cells**

In another aspect, the present invention relates to an immune effector cell expressing the chimeric antigen receptor (CAR), and includes a vector comprising a polynucleotide encoding a chimeric antigen receptor (CAR), or a polynucleotide encoding a chimeric antigen receptor (CAR).

In the present invention, the immune effector cells may be mammalian-derived cells, preferably T cells or natural killer (NK) cells.

In the present invention, the immune effector cells expressing the chimeric antigen receptor (CAR) can be prepared by introducing the CAR vector of the present invention into immune effector cells, for example, T cells or NK cells.

Specifically, CAR vector can be introduced into cells by methods known in the art, such as electroporation, lipofectamine (lipofectamine 2000, Invitrogen), and the like. For example, an immune effector cell can be transformed by a lentiviral vector to integrate the viral genome carrying the CAR molecule into the host genome to ensure long-term and stable expression of the target gene. For another example, a transposon can be used to introduce a CAR transport plasmid and a transferase transport plasmid into a target cell. For another example, a CAR molecule can be added to the genome by a gene editing method (e.g., CRISPRCas9).

In a specific embodiment of the present invention, as shown in FIG. 2, a lentiviral vector into which a polynucleotide coating CD22-CAR was inserted was prepared, and the prepared vector was transformed into T cells to prepare CD22-CAR-T cells. did. The prepared CD22-CAR-T cells express a chimeric antigen receptor targeting CD22 of the present invention.

An immune effector cell for the production of immune effector cell expressing a chimeric antigen receptor (CAR) can be obtained from a subject, wherein the "subject" includes a living organism (e.g., a mammal from which an immune response can be elicited). Examples of subjects include humans, dogs, cats, mice, rats, and transgenic species thereof. T cells can be obtained from numerous sources, including peripheral blood mononuclear cells, bone marrow, lymph node tissue, umbilical cord blood, thymus tissue, tissue from the site of infection, ascites, pleural effusion, splenic tissue, and tumors.

Such T cells can be obtained from blood units collected from a subject using any of a number of techniques known to those of ordinary skill in the art, for example, Ficoll™ isolation. Cells from blood are obtained by apheresis, and apheresis products typically contain T cells, monocytes, granulocytes, lymphocytes including B cells, other nucleated leukocytes, red blood cells, and platelets.

Cells collected by apheresis can be washed to remove the plasma fraction and place the cells in an appropriate buffer or medium for subsequent processing steps. T cells are isolated from peripheral blood lymphocytes by lysing red blood cells and depleting monocytes, for example by centrifugation through a PERCOLL™ gradient or by countercurrent centrifugation.

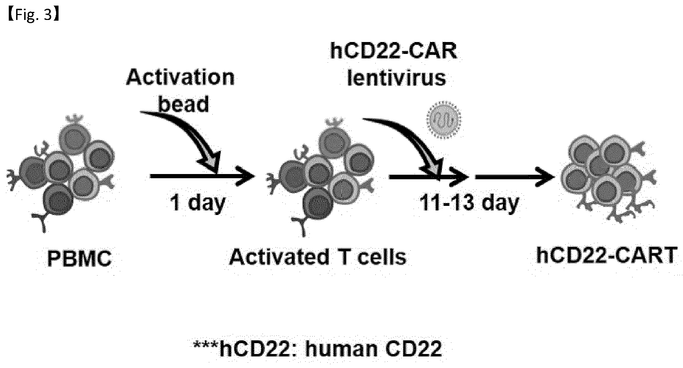

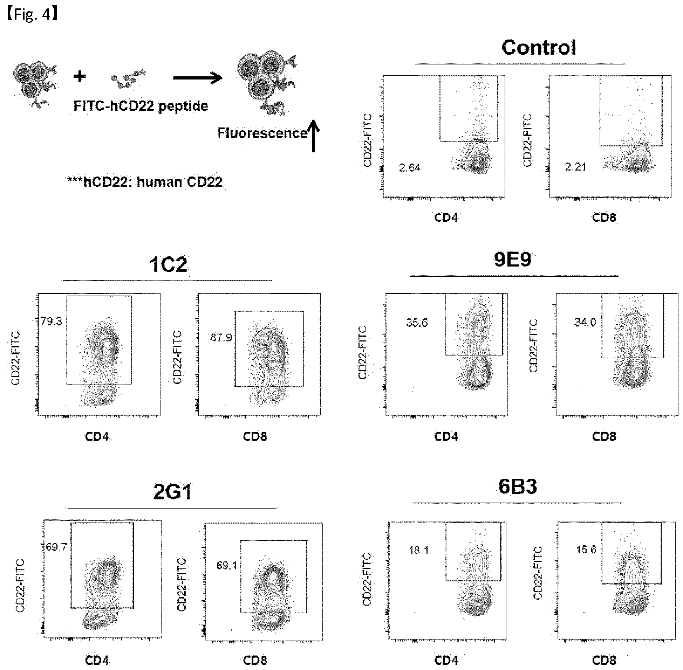

In a specific embodiment of the present invention, as shown in FIG. 3, activated T cells were isolated from peripheral blood mononuclear cells (PBMCs), and then CD22-CAR lentivirus was transduced into T cells to prepare CD22-CAR-T cells. As a result of confirming CD22-peptide binding ability of the prepared CD22-CAR-T cells, it was confirmed that the CD22-CAR-T cells expressing CD4 or CD8 increased as the CD22-peptide increased, as shown in FIG. 4. This means that the CD22-CAR-T cells prepared in the present invention effectively bind to CD22.

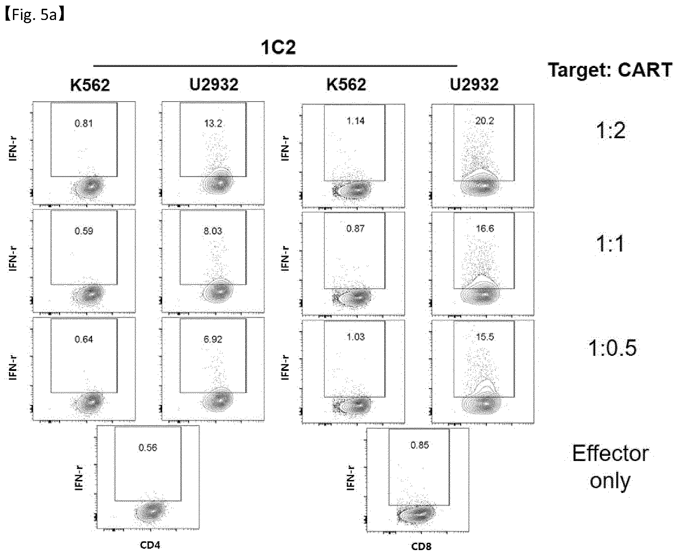

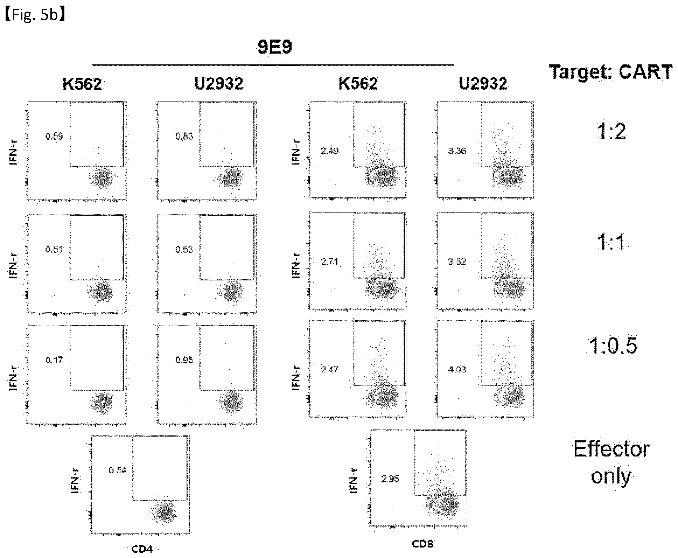

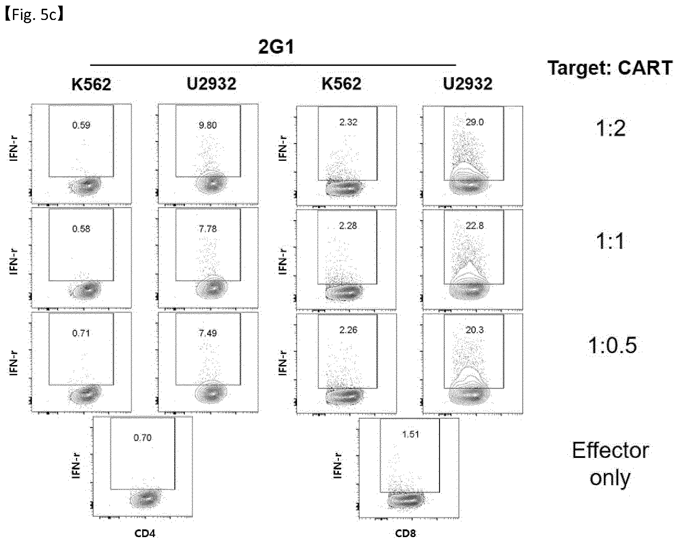

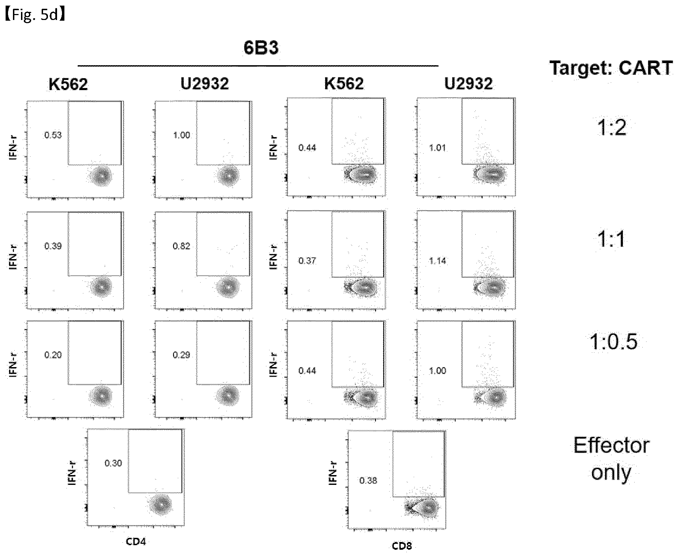

In another specific embodiment of the present invention, in order to confirm activation of CD22-CAR-T cells, the level of IFNy expression by CD22-CAR-T cells in the presence of target cells was checked. As a result, as shown in Figs. 5a to 5d, T cells were not activated in K562 cells that do not express CD22, whereas T cells were activated in the presence of U2932 cells expressing CD22, thereby increasing IFNy expression.

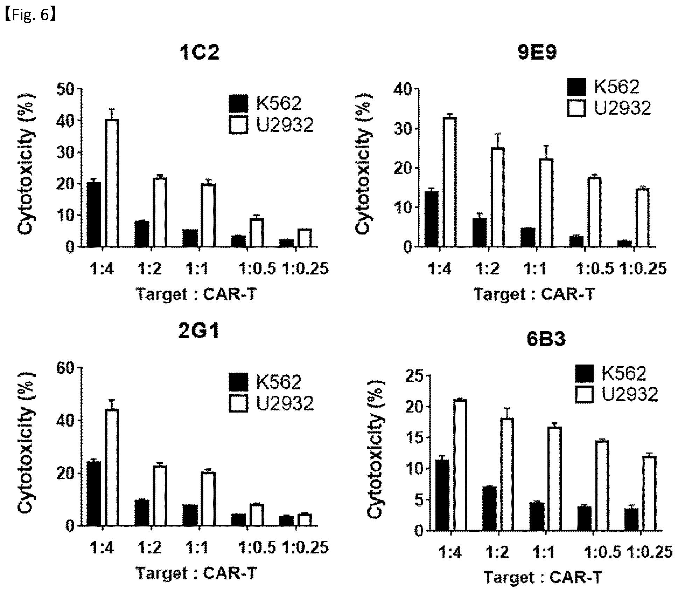

In another specific embodiment of the present invention, as a result of confirming the killing effect of the target cells by the CD22-CAR-T cells, as shown in Fig. 6, it was confirmed that CD22-CAR-T cells showed the killing effect on U2932 cells expressing CD22.

That is, the chimeric antigen receptor and CAR-T cell targeting CD22 of the present invention can be usefully used as a composition for preventing or treating diseases related to B cells or CD22 expression.

## Composition for preventing or treating diseases mediated by CD22 expression

In another aspect, the present invention provides a pharmaceutical for the prevention or treatment of diseases mediated by CD22-expressing cells, comprising: the antibody specifically binding to CD22 or the fragment thereof; or the immune effector cell expressing a chimeric antigen receptor targeting CD22.

In the present invention, the disease mediated by cells expressing CD22 may be any one of lymphoma, non-Hogkins lymphoma (NHL), aggressive NHL, relapsed aggressive NHL, relapsed delayed NHL, refractory NHL, refractory Delayed NHL, chronic lymphocytic leukemia (CLL), small lymphoma, leukemia, hairy cell leukemia (HCL), acute lymphocytic leukemia (ALL), Burkitt's lymphoma and mantle cell lymphoma.

In the present invention, the composition may include a therapeutic agent for a disease mediated by cells expressing CD22, wherein the therapeutic agent may be covalently bound to the heavy and/or light chain of antibody that specifically binds to CD22, it can be administered in combination with antibody or CD22-CAR-T cell specific for CD22 of the present invention

The therapeutic agent includes a small molecule drug, a peptide drug, a toxin (e.g., a cytotoxin), and the like.

The low molecular weight drug exhibits a pharmaceutical activity of interest, and generally may be a compound having a molecular weight of about 800 Da or less or 2000 Da or less. A small inorganic molecule refers to a molecule containing no carbon atoms, while an organic small molecule refers to a compound containing at least one carbon atom.

The peptide drug refers to amino acids containing polymeric compounds, including naturally occurring and non-naturally occurring peptides, oligopeptides, cyclic peptides, polypeptides and proteins, as well as peptide mimics. The peptide drug may be obtained by chemical synthesis or produced from a genetically encoded source (e.g., a recombinant source). The molecular weight of the peptide drug may range from 200 Da to 10 kDa or more.

The toxin is preferably a cytotoxin, and includes, but is not limited to, ricin, abrin, diphtheria toxin, Pseudomonas exotoxin (e.g., PE35, PE37, PE38, PE40, etc.), saporin, gelonin, pokeweed antivirul protein (PAP), botulinum toxin, bryodin, momordin and/or buganin.

In addition, the therapeutic agent may be an anticancer agent. Anticancer agents reduce the proliferation of cancer cells and include non-peptidyl (i.e., non-protein) compounds, including cytotoxic agents and cytostatic agents. Non-limiting examples of anticancer agents include alkylating agents, nitrosourea, antimetabolites, antitumor antibiotics, plant (vinca) alkaloids, and steroid hormones. Peptide compounds may also be used.

In the pharmaceutical composition, the antibody that specifically binds to CD22 or an immune effector cell expressing a chimeric antigen receptor targeting CD22 may be the only active ingredient in the composition for treatment or diagnosis, or, can be used with other active ingredients for example, an anti-T cell, other antibody components such as anti-IFNy or anti-LPS antibody, or non-antibody components such as xanthine.

The pharmaceutical composition preferably contains a therapeutically effective amount of antibody of the present invention. As used herein, the term "therapeutically effective amount" refers to an amount of a therapeutic agent required to treat, ameliorate, or prevent a target disease or condition, or the amount of a therapeutic agent required to exhibit a detectable therapeutic or prophylactic effect. For any antibody, a therapeutically effective dose can be initially determined by cell culture assays or animal models, usually rodents, rabbits, dogs, pigs, or primates. Animal models can also be used to determine appropriate concentration ranges and routes of administration. Such information can be used to determine useful dosages and routes for dosing in humans.

The precise effective amount for a human patient can vary depending on the severity of the disease state, the patient's general health, the patient's age, weight and sex, diet, administration time, administration frequency, drug composition, response sensitivity, and tolerance/response to treatment. The amount can be determined by routine experimentation and is within the scope of the clinician's judgment. In general, an effective dosage is 0.01-50 mg/kg, preferably 0.1-20 mg/kg, more preferably about 15 mg/kg.

The compositions may be administered to the patient individually or in combination with other preparations, agents, or hormones.

The dosage at which the antibody of the present invention is administered depends on the nature of the condition to be treated, the grade of malignant lymphoma or leukemia, and whether the antibody is used to prevent disease or to treat an existing condition.

The frequency of administration depends on the half-life of the antibody molecule and the duration of the drug's effect. If an antibody molecule has a short half-life (e.g., 2 to 10 hours), it may be necessary to provide one or more doses per day. Alternatively, if an antibody molecule has a long half-life (e.g., 2 to 15 days), it may be necessary to provide a dose once a day, once a week, or once every 1 or 2 months.

In addition, the pharmaceutical composition may contain a pharmaceutically acceptable carrier for administration of an antibody. The carrier itself must not cause the production of an antibody that is harmful to the subject receiving the composition, and must be non-toxic. Suitable carriers may be slowly metabolized macromolecules, such as proteins, polypeptides, liposomes, polysaccharides, polylactic acid, polyglycolic acid, amino acid polymers, amino acid copolymers and inactive viral particles.

A pharmaceutically acceptable salt may be used, and it contains for example, mineral acid salts such as hydrochloride, hydrobromide, phosphate and sulfate, or salts of organic acids such as acetic acid, propionic acid, malonic acid and benzoic acid.

A pharmaceutically acceptable carrier in therapeutic compositions may additionally include liquids such as water, saline, glycerol and ethanol. Additionally, auxiliary substances such as wetting agents, emulsifying agents or pH buffering agents may be present in such compositions. The carrier may be formulated as tablets, pills, sugar-coated tablets, capsules, liquids, gels, syrups, slurries and suspensions for ingestion of the pharmaceutical composition by a patient.

Preferred forms for administration may include those suitable for parenteral administration, for example by injection or infusion. When the product is intended for infusion or injection, it may take the form of suspensions, solutions or emulsions in oil or water-soluble excipients, which may contain prescription agents such as suspending, preservative, stabilizing and/or dispersing agents. Alternatively, an antibody molecule may be in anhydrous form and reconstituted with an appropriate sterile solution prior to use.

Once formulated, the compositions of the present invention can be administered directly to a patient. The patients to be treated may be animals. However, the composition is preferably adapted for administration to human patients.

The pharmaceutical composition of the present invention is not limited, but oral, intravenous, intramuscular, intraarterial, intramedullary, intrathecal, intraventricular, transdermal, transcutaneous, subcutaneous, intraperitoneal, intranasal, intestinal, topical, sublingual, intravaginal or rectal routes. Typically, the therapeutic composition may be prepared as injectable forms as liquid solutions or suspensions. In addition, solid forms suitable for solution or suspension in liquid excipients prior to injection may be prepared.

Direct delivery of the composition may generally be achieved by injection, subcutaneous injection, intraperitoneal injection, intravenous injection, intramuscular injection, or may be delivered to the interstitial space of a tissue. In addition, the composition may be administered to the wound site. Dosage treatment may be a single dose schedule or a multiple dose schedule.

The active ingredient in the composition may be an antibody molecule. As such, it may be susceptible to degradation in the gastrointestinal tract. Thus, if the composition is administered by a route using the gastrointestinal tract, the composition will need to contain an agent that protects the antibody from degradation but releases the antibody once absorbed from the gastrointestinal tract.

A complete discussion of pharmaceutically acceptable carriers is available in Remington's Pharmaceutical Sciences (Mack Publishing Company, NJ, 1991).

## Diagnosis or monitoring of diseases mediated by cells expressing CD22

In another aspect, the present invention relates to a composition for diagnosing or monitoring a disease mediated by cells expressing CD22, comprising the antibody that specifically binds to CD22.

The antibody that specifically binds to CD22 may be directly or indirectly labeled. An indirect label includes a secondary antibody comprising a detectable label, wherein the secondary antibody binds to an antibody that specifically binds to CD22. Another indirect label includes biotin, wherein an antibody that specifically binds to biotinylated CD22 can be detected using avidin or streptavidin containing a detectable label.

A suitable detectable label includes any composition detectable by spectroscopic, photochemical, biochemical, immunochemical, electrical, optical or chemical means. A suitable labels includes, but are not limited to, magnetic beads, fluorescent dyes (e.g., fluorescein isothiocyanate, Texas red, rhodamine, green fluorescent protein, red fluorescent protein, yellow fluorescent protein, etc.), radioactive labels (e.g., For example, $^3$H, $^{125}$I, $^{35}$S, $^{14}$C or $^{32}$P), enzymes (e.g., mustard radish peroxidase, alkaline phosphatase, luciferase and the ones commonly used for enzyme-linked immunosorbent assay (ELISA)) and colorimetric labels such as colloidal gold or tinted glass or plastic (e.g., polystyrene, polypropylene, latex, etc.) beads.

In addition, for diagnosis or monitoring, the antibody may be labeled with a fluorescent protein, and may contain a contrast agent or a radioisotope.

When the antibody that specifically binds to CD22 of the present invention is used in a diagnostic kit, the antibody is immobilized on a support, and the support may be a microplate, microarray, chip, glass, bead or particle, or a membrane.

Hereinafter, preferred examples are presented to help the understanding of the present invention. However, the following examples are only provided for easier understanding of the present invention, and the contents of the present invention are not limited by the following examples.

## Example 1: Preparation and selection of antibodies that specifically bind to CD22

To select the antibody specific for CD22 peptide, a hybridoma producing the antibody binding to CD22 was prepared and the antibody was selected.

First, splenocytes were extracted by immunization with CD22 protein (Acrobiosystems Inc., cat#CD2-H52H8, USA), and hybridoma cells were prepared through cell fusion with mouse myeloma cells and splenocytes.

Mouse myeloma cells used for cell fusion cannot survive in HAT medium because they do not have HGPRT (HypoxanthineGuanidine-Phosphoribosyl-Transferase), but hybridomas can survive in HAT medium by fusion with splenocytes. Since only hybridomas could be grown using this, it was usually grown in HAT medium until hybridomas were established.

The limiting dilution method was used to select hybridomas producing an antibody binding to CD22 from among the proliferated hybridomas. First, it was made to be less than one cell per 96 well, and then, it was confirmed by ELISA whether the antibody obtained from the clones proliferated from one cell binds to CD22, and clones that bind to CD22 were selected. The above process was repeated three times to select hybridomas producing an antibody binding to CD22. In this way, five kinds of antibodies that bind to CD22 were obtained.

The five antibodies were named 1C2, 9E9, 2A9, 2G1 and 6B3, respectively, and their nucleotide and amino acid sequences were analyzed. The sequence information for the heavy chain variable region and the light chain variable region of each antibody according to the sequencing results was shown in Tables 1 to 5, and the underlined parts in Tables 1 to 5 are complementarity determining region (CDR).

[Table 1]

| Sequence information of 1C2 antibody | | |
|---|---|---|
| 1C2 | sequence information | SEQ ID NO: |
| Heavy chain variable region CDR1 | GYTFTSYWMNW | SEQ ID NO: 1 |
| Heavy chain variable region CDR2 | IDPSDSET | SEQ ID NO: 2 |
| Heavy chain variable region CDR3 | ARWGNYDYDVWAMDY | SEQ ID NO: 3 |
| Light chain variable region CDR1 | QNIVHLNGNTF | SEQ ID NO: 4 |
| Light chain variable region CDR2 | KVS | SEQ ID NO: 5 |
| Light chain variable region CDR3 | FQGSHVPYT | SEQ ID NO: 6 |
| amino acid sequence of heavy chain variable | QVQLQQPGAELVRPGASVKLSCKAS **GYTFTSYWMNW** VKQRPGQGLEWIGM **IDPSDSET** HYNQMFKDKATLTVDKSSSTAYMQLSSLTSEDSAVYY | SEQ ID NO: 7 |
| region | C **ARWGNYDYDVWAMDY** WGQGTSVTVSS | |
| amino acid sequence of light chain variable region | DVLMTQTPLSLPVSLGDQASISCRSS **QNIVHLNGNTF** LEWFLQKPGQSPKLLIY **KVS** NRFSGVPDRFSGSGSGTDFTLKISRVEAEDLGVYYC **FQGSHVPYT** FGGGTKLEIK | SEQ ID NO: 8 |
| nucleotide sequence of heavy chain variable region | CAGGTGCAGCTGCAGCAGCCCGGCGCCGAGCTGG TGAGGCCCGGCGCCAGCGTGAAGCTGAGCTGCAA GGCCAGCGGCTACACCTTCACCAGCTACTGGATGA ACTGGGTGAAGCAGAGGCCCGGCCAGGGCCTGGA GTGGATCGGCATGATCGACCCCAGCGACAGCGAG ACCCACTACAACCAGATGTTCAAGGACAAGGCCAC CCTGACCGTGGACAAGAGCAGCAGCACCGCCTACA T GCAGCTGAGCAGCCTGACCAGCGAGGACAGCGCC GTGTACTACTGCGCCAGGTGGGGCAACTACGACTA CGACGTGTGGGCCATGGACTACTGGGGCCAGGGC ACCAGCGTGACCGTGAGCAGC | SEQ ID NO: 9 |

(continued)

| Sequence information of 1C2 antibody | | |
|---|---|---|
| 1C2 | sequence information | SEQ ID NO: |
| nucleotide sequence of light chain variable region | GACGTGCTGATGACCCAGACCCCCCTGAGCCTGCC CGTGAGCCTGGGCGACCAGGCCAGCATCAGCTGCA GGAGCAGCCAGAACATCGTGCACCTGAACGGCAAC ACCTTCCTGGAGTGGTTCCTGCAGAAGCCCGGCCA GAGCCCCAAGCTGCTGATCTACAAGGTGAGCAACA GGTTCAGCGGCGTGCCCGACAGGTTCAGCGGCAG CGGCAGCGGCACCGACTTCACCCTGAAGATCAGCA GGGTGGAGGCCGAGGACCTGGGCGTGTACTACTG CTTCCAGGGCAGCCACGTGCCCTACACCTTCGGCG GCGGCACCAAGCTGGAGATCAAG | SEQ ID NO: 10 |

[Table 2]

| Sequence information of 9E9 antibody | | |
|---|---|---|
| 9E9 | sequence information | SEQ ID NO: |
| Heavy chain variable region CDR1 | GYAFSIYW | SEQ ID NO: 11 |
| Heavy chain variable region CDR2 | IYPGDGDT | SEQ ID NO: 12 |
| Heavy chain variable region CDR3 | ARRGITPWFAY | SEQ ID NO: 13 |
| Light chain variable region CDR1 | QSLLNSSNQKNY | SEQ ID NO: 14 |
| Light chain variable region CDR2 | FAS | SEQ ID NO: 15 |
| Light chain variable region CDR3 | CQQHYSTPLT | SEQ ID NO: 16 |
| amino acid sequence of heavy chain variable region | EVQLEESGAELVRPGSSVKISCKAS GYAFSIYW MNWVKQRPGQGLEWIGQ **IYPGDGDT** NYNGKFKGKATLTADKSSSTAYMQLSSLTSEDSAVYF C **ARRGITPWFAY** WGQGTLVTVSA | SEQ ID NO: 17 |
| amino acid sequence of light chain variable region | DIVLTQSPSSLAMSVGQKVTMSCKSS **QSLLNSSNQKNY** LAWYQQKPGQSPKLLVY **FAS** TRESGVPDRFIGSGSGTDFTLTISSVQAEDLADYF **CQQHYSTPLT** FGAGTKLELK | SEQ ID NO: 18 |

(continued)

| Sequence information of 9E9 antibody | | |
|---|---|---|
| 9E9 | sequence information | SEQ ID NO: |
| nucleotide sequence of heavy chain variable region | GAGGTTCAGCTGGAGGAGTCTGGGGCTGAGCTGGTGAGGCCTGGGTCCTCAGTGAAGATTTCCTGCAAGGCTTCTGGCTATGCATTCAGTATCTACTGGATGAACTGGGTGAAGCAGAGGCCTGGACAGGGTCTTGAGTGGATTGGACAGATTTATCCTGGAGATGGTGATACTAACTACAATGGAAAGTTCAAGGGTAAAGCCACACTGACTGCAGACAAATCCTCCAGCACAGCCTACATGCAGCTCAGCAGCCTAACATCTGAGGACTCTGCGGTCTATTTCTGTGCAAGGCGGGGGATTACGCCCTGGTTTGCTTACTGGGGCCAAGGGACTCTGGTCACTGTCTCTGCA | SEQ ID NO: 19 |
| nucleotide sequence of light chain variable region | GACATTGTGCTGACACAGTCTCCATCCTCCCTGGCTATGTCAGTAGGACAGAAGGTCACTATGAGCTGCAAGTCCAGTCAGAGCCTTTTAAATAGTAGCAATCAAAAGAACTATTTGGCCTGGTACCAGCAGAAACCAGGACAGTCTCCTAAACTTCTGGTATACTTTGCATCCACTAGGGAATCTGGGGTCCCTGATCGCTTCATAGGCAGTGGATCTGGGACAGATTTCACTCTTACCATCAGCAGTGTGCAGGCTGAAGACCTGGCAGATTACTTCTGTCAGCAACATTATAGCACTCCGCTCACGTTCGGTGCTGGGACCAAGCTGGAGCTGAAA | SEQ ID NO: 20 |

[Table 3]

| Sequence information of 2A9 antibody | | |
|---|---|---|
| 2A9 | sequence information | SEQ ID NO: |
| Heavy chain variable region CDR1 | GYTFTTYP | SEQ ID NO: 21 |
| Heavy chain variable region CDR2 | FHPYNDDT | SEQ ID NO: 22 |
| Heavy chain variable region CDR3 | ARGDYYVSSWYFDV | SEQ ID NO: 23 |
| Light chain variable region CDR1 | QNVGTN | SEQ ID NO: 24 |
| Light chain variable region CDR2 | SAS | SEQ ID NO: 25 |
| Light chain variable region CDR3 | QQYNSYPLT | SEQ ID NO: 26 |
| amino acid sequence of heavy chain variable region | EVQLEESGAELVKPGASVKMSCKAF **GYTFTTYP** IEWMKQNHGKSLEWIGN **FHPYNDDT** KYNEKFKGKAKLTVEKSSSTVYLELSRLTSDDSAVYYC **ARGDYYVSSWYFDV** WGAGTTVTVSS | SEQ ID NO: 27 |

(continued)

| Sequence information of 2A9 antibody | | |
|---|---|---|
| 2A9 | sequence information | SEQ ID NO: |
| amino acid sequence of light chain variable region | DIVMTQSQKFMSTSVGDRVSVTCKAS **QNVGTN** VAWYQQKPGQSPKALIY **SAS** YRYSGVPDRFTGSGSGTDFTLTISNVQSEDLAEYFC **QQYNSYPLT** FGSGTKLEIK | SEQ ID NO: 28 |
| nucleotide sequence of heavy chain variable region | GAGGTGCAGCTGGAGGAGAGCGGCGCCGAGCTG GTGAAGCCCGGCGCCAGCGTGAAGATGAGCTGCA AGGCCTTCGGCTACACCTTCACCACCTACCCCATCG AGTGGATGAAGCAGAACCACGGCAAGAGCCTGGA GTGGATCGGCAACTTCCACCCCTACAACGACGACA CCAAGTACAACGAGAAGTTCAAGGGCAAGGCCAA GCTGACCGTGGAGAAGAGCAGCAGCACCGTGTAC CTGGAGCTGAGCAGGCTGACCAGCGACGACAGCG CCGTGTACTACTGCGCCAGGGGCGACTACTACGTG AGCAGCTGGTACTTCGACGTGTGGGGCGCCGGCAC CACCGTGACCGTGAGCAGC | SEQ ID NO: 29 |
| nucleotide sequence of light chain variable region | GACATCGTGATGACCCAGAGCCAGAAGTTCATGAG CACCAGCGTGGGCGACAGGGTGAGCGTGACCTGC AAGGCCAGCCAGAACGTGGGCACCAACGTGGCCT GGTACCAGCAGAAGCCCGGCCAGAGCCCCAAGGC CCTGATCTACAGCGCCAGCTACAGGTACAGCGGCG TGCCCGACAGGTTCACCGGCAGCGGCAGCGGCACC GACTTCACCCTGACCATCAGCAACGTGCAGAGCGA GGACCTGGCCGAGTACTTCTGCCAGCAGTACAACA GCTACCCCCTGACCTTCGGCAGCGGCACCAAGCTG GAGATCAAG | SEQ ID NO: 30 |

[Table 4]

| Sequence information of 2G1 antibody | | |
|---|---|---|
| 2G1 | sequence information | SEQ ID NO: |
| Heavy chain variable region CDR1 | GFSLTSYDI | SEQ ID NO: 31 |
| Heavy chain variable region CDR2 | IWTGGGT | SEQ ID NO: 32 |
| Heavy chain variable region CDR3 | VPHYYGYAMDYW | SEQ ID NO: 33 |
| Light chain variable region CDR1 | Qdinky | SEQ ID NO: 34 |
| Light chain variable region CDR2 | YTS | SEQ ID NO: 35 |

(continued)

| Sequence information of 2G1 antibody | | |
|---|---|---|
| 2G1 | sequence information | SEQ ID NO: |
| Light chain variable region CDR3 | LQYDNLLLT | SEQ ID NO: 36 |
| amino acid sequence of heavy chain variable region | EVQLQESGPGLVAPSQSLSITCTVS **GFSLTSYDI** SWIRQPPGKGLEWLGV **IWTGGGT** NYNSAFMSRLSISKDNSKSQVFLKMNSLQTDDTAIYY C **VPHYYGYAMDYW** GQGTSVTVSS | SEQ ID NO: 37 |
| amino acid sequence of light chain variable region | DIVLTQSPSSLSASLGGKVTITCKAS **QDINKY** IAWYQHKPGKGPRLLIH **YTS** TLQPGIPSRFSGSGSGRDYSFSISNLEPEDIATYYC **LQYDNLLT** FGAGTKLELK | SEQ ID NO: 38 |
| nucleotide sequence of heavy chain variable region | GAGGTGCAGCTGCAGGAGTCAGGACCTGGCCTGG TGGCGCCCTCACAGAGCCTGTCCATTACCTGCACTG TCTCTGGGTTCTCATTAACCAGCTATGATATAAGCT GGATTCGCCAGCCACCAGGAAAGGGTCTGGAGTG GCTTGGAGTAATATGGACTGGTGGAGGCACAAATT ATAATTCAGCTTTCATGTCCAGACTGAGCATCAGCA AGGACAACTCCAAGAGCCAAGTTTTCTTAAAAATG AACAGTCTGCAAACTGATGACACAGCCATATATTAC TGTGTCCCTCATTACTACGGCTATGCTATGGACTAC TGGGGTCAAGGAACCTCAGTCACCGTCTCCTCA | SEQ ID NO: 39 |
| nucleotide sequence of light chain variable region | GATATTGTGCTGACCCAGTCTCCATCCTCACTGTCT GCATCTCTGGGAGGCAAAGTCACCATCACTTGCAA GGCAAGCCAAGACATTAACAAGTATATAGCTTGGT ACCAACACAAGCCTGGAAAAGGTCCTAGGCTGCTC ATACATTACACATCTACATTACAGCCAGGCATCCCA TCAAGGTTCAGTGGAAGTGGGTCTGGGAGAGATT ATTCCTTCAGCATCAGCAACCTGGAGCCTGAAGATA TTGCAACTTATTATTGTCTACAGTATGATAATCTGCT CACGTTCGGTGCTGGGACCAAGCTGGAGCTGAAA | SEQ ID NO: 40 |

[Table 5]

| Sequence information of 6B3 antibody | | |
|---|---|---|
| 6B3 | sequence information | SEQ ID NO: |
| Heavy chain variable region CDR1 | GYTFTDYN | SEQ ID NO: 41 |
| Heavy chain variable region CDR2 | IYPYNGGT | SEQ ID NO: 42 |
| Heavy chain variable region CDR3 | ARLYYYGRTSYWG | SEQ ID NO: 43 |

(continued)

| Sequence information of 6B3 antibody | | |
| --- | --- | --- |
| 6B3 | sequence information | SEQ ID NO: |
| Light chain variable region CDR1 | QNVGTY | SEQ ID NO: 44 |
| Light chain variable region CDR2 | SAS | SEQ ID NO: 45 |
| Light chain variable region CDR3 | HQYDSYPYT | SEQ ID NO: 46 |
| amino acid sequence of heavy chain variable region | EVQLQQSGPELVKPGASVKISCKAS **GYTFTDYN** MHWVKQSHGKSLEWIGY **IYPYNGGT** GYKQKFKSKATLTVDNSSSTAYMELRSLTSEDSAVYYC **ARLYYYGRTSYWG** QGTTLTVSS | SEQ ID NO: 47 |
| amino acid sequence of light chain variable region | DIVMTQSQKFMSTSVGDRVSVTCKAS **QNVGTY** VAWYQQKPGQSPKALIY **SAS** YRYSGVPDRFTGSGSGTDFTLTISIVQSEDLAEYFC **HQYDSYPYT** FGGGTKLEI | SEQ ID NO: 48 |
| nucleotide sequence of heavy chain variable region | GAGGTGCAGCTGCAGCAGAGCGGCCCCGAGCTGG TGAAGCCCGGCGCCAGCGTGAAGATCAGCTGCAA GGCCAGCGGCTACACCTTCACCGACTACAACATGC ACTGGGTGAAGCAGAGCCACGGCAAGAGCCTGGA GTGGATCGGCTACATCTACCCCTACAACGGCGGCA CCGGCTACAAGCAGAAGTTCAAGAGCAAGGCCACC CTGACCGTGGACAACAGCAGCAGCACCGCCTACAT GGAGCTGAGGAGCCTGACCAGCGAGGACAGCGCC GTGTACTACTGCGCCAGGCTGTACTACTACGGCAG GACCAGCTACTGGGGCCAGGGCACCACCCTGACCG TGAGCAGC | SEQ ID NO: 49 |
| nucleotide sequence of light chain variable region | GACATCGTGATGACCCAGAGCCAGAAGTTCATGAG CACCAGCGTGGGCGACAGGGTGAGCGTGACCTGC AAGGCCAGCCAGAACGTGGGCACCTACGTGGCCTG GTACCAGCAGAAGCCCGGCCAGAGCCCCAAGGCCC TGATCTACAGCGCCAGCTACAGGTACAGCGGCGTG CCCGACAGGTTCACCGGCAGCGGCAGCGGCACCG ACTTCACCCTGACCATCAGCATCGTGCAGAGCGA | SEQ ID NO: 50 |
| | GGACCTGGCCGAGTACTTCTGCCACCAGTACGACA GCTACCCCTACACCTTCGGCGGCGGCACCAAGCTG GAGATCAAG | |

## Example 2: Confirmation of specificity of the selected antibody for CD22 - ELISA analysis

In the present invention, in order to confirm the specificity of 1C2, 9E9, 2A9, 2G1 and 6B3 antibodies established in Example 1 for CD22, ELISA analysis was performed.

First, in order to encode the CD22 peptide, CD22-His tag (CD22 extracellular domain; Acrobiosystems Inc.) was dispensed in a 96-well plate at a concentration of 100 ng/well, and then reacted at 4°C overnight. Then, after treatment with 1 X PBST containing 3% BSA, blocking at room temperature for 30 minutes.

1C2, 9E9, 2A9, 2G1 and 6B3 antibodies were treated in each well, and then reacted at room temperature for 2 hours, and then washed 3 times with 1 X PBST. Secondary antibody (anti-HRP, 1:10,000) was treated and reacted at room temperature for 30 minutes, washed 3 times with 1X PBST, and then treated with TMB for color development and reacted at room temperature for 5 minutes. Finally, the reaction was terminated by treatment with a stop solution of 1N $H_2SO_4$, and then the absorbance was measured at 450 nm.

[Table 6]

| ELISA Experimental Conditions | |
|---|---|
| Immobilization | Corning (Cat#. 3690) |
| Antigen | CD22-His tag, 100 ng/well |
| Primary Ab | 0.4 µg/ml |
| Secondary Ab-HRP | 1:10,000 |
| TMB substrate incubation | 5 min |
| Measurement filter | 450nm |
| plate reader | Infinite F50 |

[Table 7]

| Results of ELISA test | |
|---|---|
| Antibody type | OD450 measurements |
| 1C2 | 2.4289 |
| 9E9 | 1.7581 |
| 2A9 | 2.9304 |
| 2G1 | 2.5395 |
| 6B3 | 1.7930 |

As a result, as shown in Table 7, it was confirmed that all of the antibodies selected in the present invention specifically bound to CD22.

**Example 3: Confirmation of specificity for CD22 of the selected antibody - FACS analysis**

In the present invention, in order to confirm the specificity of the 1C2, 9E9, 2A9, 2G1 and 6B3 antibodies established in Example 1 for CD22, FACS analysis was performed.

First, $1\times 10^6$ CD22-expressing non-cell lymphoma U2932 cells (B-cell lymphoma U2932 cell) and 1 µg each of 1C2, 9E9, 2A9, 2G1 and 6B3 antibodies were reacted for 30 minutes, and then surface was stained with a secondary antibody. After that, it was measured by flow cytometry.

As a positive control, PE-conjugated anti-CD22 antibody (Biolegend Inc., cat# 302506, USA) was used, and as a secondary antibody, PE-conjugated goat anti-mouse IgG (Biolegend Inc., cat# 405307, USA) was used.

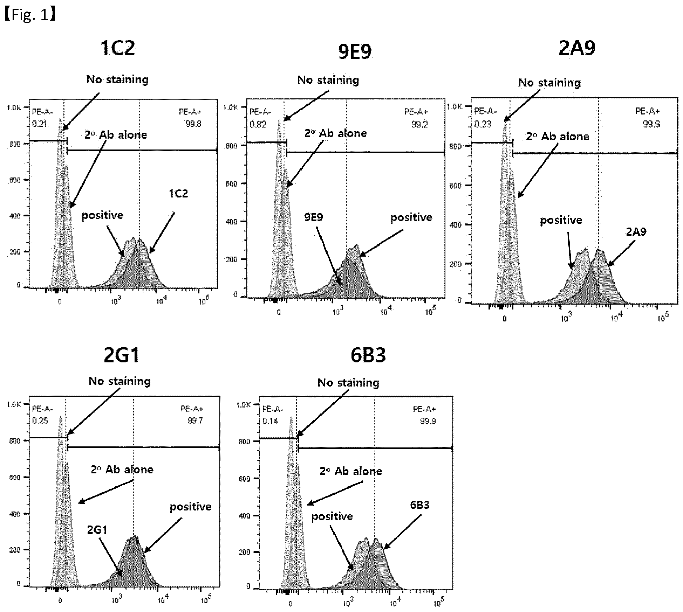

As a result, as shown in FIG. 1, it was confirmed that all of the 1C2, 9E9, 2A9, 2G1 and 6B3 antibodies specifically bound to CD22-expressing cells.

**Example 4: Construction of a chimeric antigen receptor (CAR) expression vector targeting CD22**

In the present invention, using 1C2, 9E9, 2G1 and 6B3 antibodies with high CD22 specificity among the antibodies prepared in Example 1 above, a lentiviral vector expressing a chimeric antigen receptor (CAR) targeting CD22 (CD22- CAR lentivirus) was prepared.

As shown in the schematic diagram of Figure 2, CAR DNA composed of: EFla promoter (SEQ ID NO: 63); a polynucleotide encoding a signal peptide (SEQ ID NO: 64);

a polynucleotide encoding the CD22-binding domain (7C7 antibody represented by the nucleotide sequence of SEQ ID NO: 74, 5H4 antibody represented by the nucleotide sequence of SEQ ID NO: 76, a polynucleotide encoding a CD22-binding domain (1C2 represented by SEQ ID NO: 54, 9E9 represented by SEQ ID NO: 56, or 2G1 represented by SEQ ID NO: 60 or 6B3 represented by SEQ ID NO: 62); a polynucleotide encoding the CD8 hinge region (SEQ ID NO: 65); a polynucleotide encoding a transmembrane domain (SEQ ID NO: 66); a polynucleotide encoding 4-1BB (costimulatory domain) (SEQ ID NO: 67); a polynucleotide encoding CD3ζ intracellular signal transduction domain (SEQ ID NO: 68); and a polynucleotide encoding WPRE (SEQ ID NO: 69); was synthesized in vitro and inserted into a 3rd generation lentivirus vector.

Lentiviral vectors were co-transfected into Lenti-X 293T cells with three vectors: pMDLg/pRRE (Addgene, cat# #12251), pMD2.G (Addgene, cat##12259), and pRSV-Rev (Addgene, cat##12253). After co-transfection, CD22-CAR lentivirus was produced. For co-infection, three vectors and Lenti-X 293T cells were cultured for 6 hours using Lipofectamine 3000 transfection kit (Invitrogen, cat# L3000-015) and Opti-MEM+GlutaMAX (gibco, cat# 51985-034) medium.

**Example 5: CD22-CAR-T cell preparation**

In the present invention, the CD22-CAR lentiviral vector prepared in Example 4 was transformed into T cells to prepare CD22-CAR-T cells.

Specifically, as shown in FIG. 3, after separating peripheral blood mononuclear cells (PBMC) from blood, T cell activation beads (T cell activation bead; Miltenyl Biotec, cat# 130-091-441) was used to activate T cells. CD22-CAR-T cells were prepared by transducing the activated T cells with the CD22-CAR lentivirus prepared in Example 4 into T cells, and transduction efficiency was increased using Lenti-boost-p.

CD22 peptide binding capacity of CD22-CAR-T cells was confirmed by flow cytometry. The CD22-CAR-T cells prepared above were reacted with FITC-CD22 protein with anti-CD3, anti-CD4, and anti-CD8 antibodies, and then fluorescence intensity was measured using a FACS machine. In the course of the analysis, the level of FITC expression in T cells was confirmed.

As a result, as shown in FIG. 4, it was confirmed that CD22-CAR-T cells expressing CD4 or CD8 binding to CD22 increased as the CD22 peptide increased. This means that the CD22-CAR-T cells prepared in the present invention effectively bound to CD22.

**Example 6: Confirmation of CD22-CAR-T cell activation by CD22 peptide**

In the present invention, in order to confirm that the CD22-CAR-T cells prepared in Example 5 are activated by the CD22 peptide, the level of IFNy expression by the CD22-CAR-T cells in the presence of target cells was checked.

As target cells, K562 cells that do not express CD22 (ATCC, cat#CCL-243) and U2932 cells that express CD22 (DSMZ, cat#ACC-633) were used. CD22-CAR-T cells and target cells were reacted at a ratio of 2:1, 1:1, 0.5:1, 0:1 for a certain period of time, and then stained with surface & intra antibody for FACS measurement (CD22 protein, INF-r, CD107a, CD3, CD4, CD8 staining). The level of IFNy expression of CD22-CAR-T reacted with target cells was confirmed on the basis of 0:1 (CAR-T only).

As a result, as shown in Figs. 5a to 5d, T cells were not activated in K562 cells that do not express CD22, whereas T cells were activated in the presence of U2932 cells expressing CD22, thereby increasing IFNy expression.

**Example 7: Confirmation of the killing effect of CD22-CAR-T cells on CD22-expressing cells**

In the present invention, the killing effect of target cells by CD22-CAR-T cells was confirmed.

As target cells, K562 cells, which do not express CD22, and U2932 cells, which express CD22, were used. After mixing the above cells with CD22-CAR-T cells at ration of 1:4, 1:2, 1:1, 1:0.5 and 1:0.25, luminescence (CytoTox-Glo Cytotoxicity Assay, Promega, cat# G9291) was measured. The degree of cell death was calculated using Equation 1 below as the measured value.

[Equation 1]

$$\% \text{ Cytotoxicity} = [(\text{Experimental} - \text{Effector Spontaneous} - \text{Target Spontaneous}) / (\text{Target Maximum} - \text{Target Spontaneous})] \times 100$$

- Experimental: Luminescence value derived from the medium of the target cell and CAR-T cell complex culture
- Effector Spontaneous: Luminescence value derived from the medium of CAR-T cells only
- Target Spontaneous: Luminescence value derived from the medium of target cells only
- Target Maximum: Luminescence value derived from 100% lysis of target cells (using Lysis Reagent)

As a result, as shown in FIG. 6, it was confirmed that the CD22-CAR-T cells of the present invention specifically killed U2932 cells expressing CD22.

[Industrial Applicability]

It was confirmed that the antibody selected in the present invention specifically recognized CD22-expressing cells, and the chimeric antigen receptor (CAR) and CAR-T cells targeting CD22 using the CD22-specific antibody not only effectively bound to CD22, but also activated CAR-T cells bound to CD22.

In addition, since it was confirmed that the CAR-T cells of the present invention effectively kill CD22-expressing cells, the CD22-specific antibody, the chimeric antigen receptor targeting CD22, and CAR-T cells of the present invention can be usefully used as a composition for preventing or treating diseases relating to CD22 expression.

**Claims**

1. An antibody specifically binding to CD22 or a fragment thereof, comprising:

    (a) a heavy chain variable region comprising a CDR1 region represented by the amino acid of SEQ ID NO: 1, a CDR2 region represented by the amino acid of SEQ ID NO: 2 and a CDR3 region represented by the amino acid of SEQ ID NO: 3, and a light chain variable region comprising a CDR1 region represented by the amino acid of SEQ ID NO: 4, a CDR2 region represented by the amino acid of SEQ ID NO: 5, and a CDR3 region represented by the amino acid sequence represented by SEQ ID NO: 6;
    (b) a heavy chain variable region comprising a CDR1 region represented by the amino acid of SEQ ID NO: 11, a CDR2 region represented by the amino acid of SEQ ID NO: 12 and a CDR3 region represented by the amino acid of SEQ ID NO: 13 and a light chain variable region comprising a CDR1 region represented by the amino acid of SEQ ID NO: 14, a CDR2 region represented by amino acids of SEQ ID NO: 15 and a CDR3 region represented by amino acids of SEQ ID NO: 16;
    (c) a heavy chain variable region comprising a CDR1 region represented by the amino acid of SEQ ID NO: 21, a CDR2 region represented by the amino acid of SEQ ID NO: 22 and a CDR3 region represented by the amino acid of SEQ ID NO: 23 and a light chain variable region comprising a CDR1 region represented by the amino acid of SEQ ID NO: 24, a CDR2 region represented by the amino acid of SEQ ID NO: 25 and a CDR3 region represented by the amino acid of SEQ ID NO: 26;
    (d) a heavy chain variable region comprising a CDR1 region represented by the amino acid of SEQ ID NO: 31, a CDR2 region represented by the amino acid of SEQ ID NO: 32 and a CDR3 region represented by the amino acid of SEQ ID NO: 33 and a light chain variable region comprising a CDR1 region represented by the amino acid of SEQ ID NO: 34, a CDR2 region represented by the amino acid of SEQ ID NO: 35 and a CDR3 region represented by the amino acid of SEQ ID NO: 36; or
    (e) a heavy chain variable region comprising a CDR1 region represented by the amino acid of SEQ ID NO: 41, a CDR2 region represented by the amino acid of SEQ ID NO: 42 and a CDR3 region represented by the amino acid of SEQ ID NO: 43 and a light chain variable region comprising a CDR1 region represented by the amino acid of SEQ ID NO: 44, a CDR2 region represented by the amino acid of SEQ ID NO: 45 and a CDR3 region represented by the amino acid of SEQ ID NO: 46.

2. The antibody specifically binding to CD22 or a fragment thereof of claim 1, wherein

    the antibody (a) has a heavy chain variable region represented by the amino acid of SEQ ID NO: 7 and a light chain variable region represented by the amino acid of SEQ ID NO: 8,
    the (b) antibody has a heavy chain variable region represented by the amino acid of SEQ ID NO: 17 and a light chain variable region represented by the amino acid of SEQ ID NO: 18,
    the (c) antibody has a heavy chain variable region represented by the amino acid of SEQ ID NO: 27 and a light chain variable region represented by the amino acid of SEQ ID NO: 28,
    the (d) antibody has a heavy chain variable region represented by the amino acid of SEQ ID NO: 37 and a light chain variable region represented by the amino acid of SEQ ID NO: 38, or
    the (e) antibody has a heavy chain variable region represented by the amino acid of SEQ ID NO: 47 and a light chain variable region represented by the amino acid of SEQ ID NO: 48.

3. A polynucleotide encoding an antibody or fragment thereof that specifically binds to the CD22 of claim 1 or 2.

**4.** A vector comprising a polynucleotide encoding an antibody or fragment thereof that specifically binds to the CD22 of claim 1 or 2.

**5.** A recombinant cell producing an antibody or fragment thereof that specifically binds to CD22 transformed with the vector of claim 4.

**6.** A chimeric antigen receptor (CAR) comprising: a CD22-binding domain; a transmembrane domain; a costimulatory domain; and an intracellular signal transduction domain,
wherein the CD22-binding domain is an antibody specifically binding to CD22 or a fragment thereof, comprising:

(a) a heavy chain variable region comprising a CDR1 region represented by the amino acid of SEQ ID NO: 1, a CDR2 region represented by the amino acid of SEQ ID NO: 2 and a CDR3 region represented by the amino acid of SEQ ID NO: 3, and a light chain variable region comprising a CDR1 region represented by the amino acid of SEQ ID NO: 4, a CDR2 region represented by the amino acid of SEQ ID NO: 5, and a CDR3 region represented by the amino acid sequence represented by SEQ ID NO: 6;
(b) a heavy chain variable region comprising a CDR1 region represented by the amino acid of SEQ ID NO: 11, a CDR2 region represented by the amino acid of SEQ ID NO: 12 and a CDR3 region represented by the amino acid of SEQ ID NO: 13 and a light chain variable region comprising a CDR1 region represented by the amino acid of SEQ ID NO: 14, a CDR2 region represented by amino acids of SEQ ID NO: 15 and a CDR3 region represented by amino acids of SEQ ID NO: 16;
(c) a heavy chain variable region comprising a CDR1 region represented by the amino acid of SEQ ID NO: 21, a CDR2 region represented by the amino acid of SEQ ID NO: 22 and a CDR3 region represented by the amino acid of SEQ ID NO: 23 and a light chain variable region comprising a CDR1 region represented by the amino acid of SEQ ID NO: 24, a CDR2 region represented by the amino acid of SEQ ID NO: 25 and a CDR3 region represented by the amino acid of SEQ ID NO: 26;
(d) a heavy chain variable region comprising a CDR1 region represented by the amino acid of SEQ ID NO: 31, a CDR2 region represented by the amino acid of SEQ ID NO: 32 and a CDR3 region represented by the amino acid of SEQ ID NO: 33 and a light chain variable region comprising a CDR1 region represented by the amino acid of SEQ ID NO: 34, a CDR2 region represented by the amino acid of SEQ ID NO: 35 and a CDR3 region represented by the amino acid of SEQ ID NO: 36; or
(e) a heavy chain variable region comprising a CDR1 region represented by the amino acid of SEQ ID NO: 41, a CDR2 region represented by the amino acid of SEQ ID NO: 42 and a CDR3 region represented by the amino acid of SEQ ID NO: 43 and a light chain variable region comprising a CDR1 region represented by the amino acid of SEQ ID NO: 44, a CDR2 region represented by the amino acid of SEQ ID NO: 45 and a CDR3 region represented by the amino acid of SEQ ID NO: 46.

**7.** The chimeric antigen receptor of claim 6, wherein the transmembrane domain is a protein selected from the group consisting of CD8$\alpha$, CD4, CD28, CD137, CD80, CD86, CD152 and PD1,

the costimulatory domain is a protein selected from the group consisting of CD28, 4-1BB, OX-40 and ICOS, and the intracellular signal transduction domain is a CD3$\zeta$.

**8.** The chimeric antigen receptor according to claim 6, further comprising a hinge region between a C-terminus of the CD22-binding domain and an N-terminus of the transmembrane domain.

**9.** A polynucleotide encoding the chimeric antigen receptor (CAR) of any one of claims 6-8.

**10.** A vector comprising a polynucleotide encoding the chimeric antigen receptor (CAR) of any one of claims 6 to 8.

**11.** An immune effector cell comprising the polynucleotide encoding the chimeric antigen receptor (CAR) of any one of claims 6 to 8 or the vector comprising the polynucleotide.

**12.** A pharmaceutical composition for use in preventing or treating a disease mediated by cells expressing CD22, comprising: the antibody specifically binding to CD22 or the fragment thereof of claim 1 or 2; or the immune effector cell of claim 11.

**13.** The pharmaceutical composition for use according to claim 12, wherein the disease mediated by cells expressing CD22 is any one selected from the group consisting of lymphoma, non-Hogkins lymphoma (NHL), aggressive NHL, relapsed aggressive NHL, relapsed delayed NHL, refractory NHL, Refractory delayed NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma.

14. A composition for diagnosing or monitoring a disease mediated by cells expressing CD22, comprising an antibody or fragment thereof that specifically binds to CD22 according to claim 1 or 2.

【Fig. 1】

【Fig. 2】

【Fig. 3】

***hCD22: human CD22

【Fig. 4】

【Fig. 5a】

【Fig. 5b】

【Fig. 5c】

【Fig. 5d】

【Fig. 6】

## INTERNATIONAL SEARCH REPORT

| International application No. |
| --- |
| **PCT/KR2021/004568** |

| A. | CLASSIFICATION OF SUBJECT MATTER |
| --- | --- |

**C07K 16/28**(2006.01)i; **A61P 35/00**(2006.01)i; **G01N 33/563**(2006.01)i; **G01N 33/574**(2006.01)i; **A61K 39/00**(2006.01)i

According to International Patent Classification (IPC) or to both national classification and IPC

| B. | FIELDS SEARCHED |
| --- | --- |

Minimum documentation searched (classification system followed by classification symbols)

C07K 16/28(2006.01); A61K 39/395(2006.01); A61K 47/48(2006.01); A61P 35/00(2006.01); C07K 16/30(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: CD22, 항체(antibody), 키메라 항원 수용체(CAR), 폴리뉴클레오티드 (polynucleotide), 벡터(vector)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | WO 2019-191704 A1 (EUREKA THERAPEUTICS, INC.) 03 October 2019 (2019-10-03) See abstract, claims 1-9 and 11-13, and SEQ ID NOs: 209-211 and 214-215. | 1-14 |
| A | KR 10-2018-0030656 A (UCB BIOPHARMA SPRL) 23 March 2018 (2018-03-23) See entire document. | 1-14 |
| A | KR 10-2009-0088920 A (MEDAREX, INC.) 20 August 2009 (2009-08-20) See entire document. | 1-14 |
| A | KR 10-2015-0036700 A (REDWOOD BIOSCIENCE, INC.) 07 April 2015 (2015-04-07) See entire document. | 1-14 |
| A | WO 2020-069405 A1 (NOVARTIS AG et al.) 02 April 2020 (2020-04-02) See entire document. | 1-14 |

| ☐ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **19 July 2021** | **19 July 2021** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

International application No.

**PCT/KR2021/004568**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

INTERNATIONAL SEARCH REPORT
Information on patent family members

International application No.

**PCT/KR2021/004568**

| Patent document cited in search report | | | Publication date (day/month/year) | Patent family member(s) | | | Publication date (day/month/year) |
|---|---|---|---|---|---|---|---|
| WO | 2019-191704 | A1 | 03 October 2019 | CN | 112888709 | A | 01 June 2021 |
| | | | | EP | 3774919 | A1 | 17 February 2021 |
| | | | | KR | 10-2020-0136473 | A | 07 December 2020 |
| | | | | US | 2021-0017280 | A1 | 21 January 2021 |
| KR | 10-2018-0030656 | A | 23 March 2018 | CN | 106661112 | A | 10 May 2017 |
| | | | | CN | 106661122 | A | 10 May 2017 |
| | | | | CN | 106661122 | B | 02 April 2021 |
| | | | | CN | 107849140 | A | 27 March 2018 |
| | | | | CN | 107849141 | A | 27 March 2018 |
| | | | | CN | 107922492 | A | 17 April 2018 |
| | | | | EP | 3169704 | A1 | 24 May 2017 |
| | | | | EP | 3169704 | B1 | 29 July 2020 |
| | | | | EP | 3169705 | A2 | 24 May 2017 |
| | | | | EP | 3322728 | A1 | 23 May 2018 |
| | | | | EP | 3322729 | A1 | 23 May 2018 |
| | | | | EP | 3322730 | A1 | 23 May 2018 |
| | | | | JP | 2017-522023 | A | 10 August 2017 |
| | | | | JP | 2017-529061 | A | 05 October 2017 |
| | | | | JP | 2018-524007 | A | 30 August 2018 |
| | | | | JP | 2018-524009 | A | 30 August 2018 |
| | | | | JP | 2018-529313 | A | 11 October 2018 |
| | | | | KR | 10-2017-0035930 | A | 31 March 2017 |
| | | | | KR | 10-2017-0035954 | A | 31 March 2017 |
| | | | | KR | 10-2018-0030658 | A | 23 March 2018 |
| | | | | KR | 10-2018-0030662 | A | 23 March 2018 |
| | | | | US | 10370447 | B2 | 06 August 2019 |
| | | | | US | 10590197 | B2 | 17 March 2020 |
| | | | | US | 10618957 | B2 | 14 April 2020 |
| | | | | US | 10774152 | B2 | 15 September 2020 |
| | | | | US | 2017-0204178 | A1 | 20 July 2017 |
| | | | | US | 2017-0204183 | A1 | 20 July 2017 |
| | | | | US | 2018-0201678 | A1 | 19 July 2018 |
| | | | | US | 2018-0237521 | A1 | 23 August 2018 |
| | | | | US | 2018-0273620 | A1 | 27 September 2018 |
| | | | | US | 2019-0359713 | A1 | 28 November 2019 |
| | | | | US | 2020-0024346 | A1 | 23 January 2020 |
| | | | | US | 2020-0157215 | A1 | 21 May 2020 |
| | | | | WO | 2016-009029 | A1 | 21 January 2016 |
| | | | | WO | 2016-009030 | A2 | 21 January 2016 |
| | | | | WO | 2016-009030 | A3 | 24 March 2016 |
| | | | | WO | 2017-009473 | A1 | 19 January 2017 |
| | | | | WO | 2017-009474 | A1 | 19 January 2017 |
| | | | | WO | 2017-009476 | A1 | 19 January 2017 |
| KR | 10-2009-0088920 | A | 20 August 2009 | CN | 101626782 | A | 13 January 2010 |
| | | | | CN | 101626782 | B | 27 March 2013 |
| | | | | CN | 103172743 | A | 26 June 2013 |
| | | | | CN | 103172743 | B | 08 April 2015 |
| | | | | EP | 2097097 | A2 | 09 September 2009 |
| | | | | EP | 2097097 | B1 | 30 May 2018 |
| | | | | JP | 2010-511388 | A | 15 April 2010 |

Form PCT/ISA/210 (patent family annex) (July 2019)

**INTERNATIONAL SEARCH REPORT**
**Information on patent family members**

International application No.

**PCT/KR2021/004568**

| Patent document cited in search report | | | Publication date (day/month/year) | Patent family member(s) | | | Publication date (day/month/year) |
|---|---|---|---|---|---|---|---|
| | | | | JP | 2014-039548 | A | 06 March 2014 |
| | | | | JP | 5398538 | B2 | 29 January 2014 |
| | | | | JP | 5846695 | B2 | 20 January 2016 |
| | | | | KR | 10-1552735 | B1 | 14 September 2015 |
| | | | | KR | 10-2015-0067395 | A | 17 June 2015 |
| | | | | US | 2010-0143368 | A1 | 10 June 2010 |
| | | | | US | 2013-0230530 | A1 | 05 September 2013 |
| | | | | US | 2017-0058031 | A1 | 02 March 2017 |
| | | | | US | 8481683 | B2 | 09 July 2013 |
| | | | | US | 9499632 | B2 | 22 November 2016 |
| | | | | WO | 2008-070569 | A2 | 12 June 2008 |
| | | | | WO | 2008-070569 | A3 | 20 November 2008 |
| KR | 10-2015-0036700 | A | 07 April 2015 | CN | 104582717 | A | 29 April 2015 |
| | | | | CN | 104582717 | B | 13 March 2018 |
| | | | | EP | 2874650 | A1 | 27 May 2015 |
| | | | | EP | 3539563 | A1 | 18 September 2019 |
| | | | | JP | 2015-530973 | A | 29 October 2015 |
| | | | | JP | 2019-150052 | A | 12 September 2019 |
| | | | | JP | 6578206 | B2 | 18 September 2019 |
| | | | | KR | 10-2165464 | B1 | 14 October 2020 |
| | | | | US | 10259871 | B2 | 16 April 2019 |
| | | | | US | 2014-0161870 | A1 | 12 June 2014 |
| | | | | US | 2016-0229911 | A1 | 11 August 2016 |
| | | | | US | 2019-0352395 | A1 | 21 November 2019 |
| | | | | US | 9181343 | B2 | 10 November 2015 |
| | | | | WO | 2014-014821 | A1 | 23 January 2014 |
| WO | 2020-069405 | A1 | 02 April 2020 | None | | | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 1998041641 A
- WO 1998042378 A
- WO 8810649 A
- WO 88106630 A
- WO 8807085 A
- WO 8807086 A
- WO 8809344 A
- JP 2007252372 A

**Non-patent literature cited in the description**

- **KEIRA ; MIRSTEIN et al.** *Nature,* 1975, vol. 256, 495-497
- **SAMBROOK, J. et al.** Molecular Cloning, A Laboratory Manual. Cold Spring Harbor Laboratory, 1989, vol. 1, 74
- Remington's Pharmaceutical Sciences. Mack Publishing Company, 1991